# EUROPEAN PATENT APPLICATION

(11) **EP 4 509 601 A1**
(43) Date of publication of application: **19.02.2025**
(21) Application number: 23787813.7
(22) Date of filing: 14.04.2023
(51) Int. Cl.: C12N 9/74, A61K 38/48, A61P 7/04

(54) **COAGULATION FACTOR X ACTIVATING ENZYME AND USE THEREOF**

(30) Priority: 14.04.2022 CN 202210394772
(71) Applicant: Jiangsu BioJeTay Biotechnology Co., Ltd., Wuxi City, Jiangsu 214183 (CN)
(72) Inventor: LIAO, Chuan, Beijing 100176 (CN); WANG, Qianqian, Beijing 100176 (CN); WANG, Longchao, Beijing 100176 (CN); ZHAO, Aijuan, Beijing 100176 (CN)
(74) Representative: HGF
(86) International application number: PCT/CN2023/088276
(87) International publication number: WO 2023/198171

(57) **Abstract**

Provided are a novel coagulation factor X activating enzyme, a pharmaceutical composition comprising same, and use thereof in preparing a medicament for treating hemorrhage or hemorrhagic diseases. Also provided is a method for purifying a coagulation factor X activating enzyme, which adopts a sequential combination of anion exchange chromatography and cation exchange chromatography, has higher yield while ensuring the high purity and high activity of the product, greatly improves the cost-efficiency, and is beneficial to large-scale industrial production.

## Description

### FIELD

The present application belongs to the field of biopharmaceutical technology, specifically relates to a coagulation factor X activating enzyme and its uses thereof.

### BACKGROUND

The human coagulation mechanism is a very complex and fine process, and so far, a total of 12 factors have been found to be involved in coagulation. Wherein, the coagulation factor X is one of the key components of the coagulation system. Coagulation factor X (FX) is a vitamin K-dependent serine protease, its active form (FXa) is the only physiological activating enzyme of prothrombin *in vivo,* and plays a key role in the common coagulation pathway. Coagulation factor X activating enzyme can specifically activate FX, fully expose its active site to generate FXa. FXa then forms a prothrombin complex with activated platelets, FVa, and calcium ions at the injury site, thereby increasing thrombin generation. Thrombin activates platelets and factors V and VIII at the injury site, and forms thrombus through the conversion of fibrinogen to fibrin, in order to achieve the purpose of hemostasis in bleeding patients. FX activating enzyme has potentially great application value in surgical wound bleeding, medical bleeding diseases, and coagulation system diseases, such as hemophilia.

The earliest discovered and so far the most active FX activating enzyme is isolated from the venom of the Russell's viper (Daboia russellii siamensis), the coagulation factor X activating enzyme of Russell's viper (RVV-X) is a metalloprotease which is composed of a heavy chain(α chain, which has a molecular weight of 57600 D) and two light chains (β chain, which has a molecular weight of 19400 D, and γ chain, which has a molecular weight of 16400 D) through disulfide bonds, wherein the heavy chain contains a catalytic domain, while the two light chains and C-type lectin are homologous and play a regulatory role in the calcium dependent activation process of the factor X activating enzyme. A series of post-translational modifications, such as glycosylation modification, are often performed during the synthesis of coagulation factor X activating enzyme of Russell's viper *in vivo,* and glycosylation modification may affect the enzyme activity.

A variety of methods have been known in the prior art to isolate or prepare coagulation factor X activating enzyme from Russell's viper, for example, Kisiel et al. obtained coagulation factor X activating enzyme from Russell's viper venom by Sephadex G-150 size exclusion chromatography, QAE-Sephadex A-50 anion chromatography, Sephadex G-25 size exclusion chromatography, and RVV-X with molecular weights of 90000 and 79000 were detected under non-reduced conditions respectively; a new coagulation factor X activating enzyme was obtained by subjecting Russell's viper venom sequentially to DEAE-Sephadex A-50 anion chromatography, dialysis, cellulose DEAE-32 anion chromatography and gel filtration chromatography in CN101104847A; coagulation factor X activating enzymes with molecular weights of 79KD and 98KD was obtained by subjecting Russell's viper venom sequentially to size exclusion chromatography and weak cation chromatography in CN1096523988A.

The present application provides a novel coagulation factor X activating enzyme of Russell's viper venom. In addition, the present application provides a method of isolating a factor X activating enzyme which enables the product to have high purity and high activity and significantly improves its yield for large-scale industrial production. The technical solution of the present application is of great importance for stopping bleeding or treatment of the bleeding disorders.

### CONTENT OF THE APPLICATION

### Coagulation factor X activating enzyme

In one aspect, the present application provides a novel coagulation factor X activating enzyme comprising three polypeptide chains: α, β and γ, the amino acid sequence of the α chain is set forth in SEQ ID NO: 8, the amino acid sequence of the β chain is set forth in SEQ ID NO: 10, and the amino acid sequence of the γ chain is set forth in SEQ ID NO: 12.

In some embodiments, the α, β and/or γ polypeptide chain has glycosylation modifications.

In some embodiments, at least one amino acid residue of the amino acid sequence of the α, β and/or γ polypeptide chain is covalently linked to an N-glycan.

In some embodiments, the amino acid residue is asparagine (Asn) residue.

The asparagine (Asn) residue site is selected from one or more of the following:
α chain: Asn28, Asn69, Asn163, Asn183;
β chain: Asn59; and/or
γ chain: Asn24.

In some embodiments according to any one of the coagulation factor X activating enzymes described above :
N-glycans at the Asn28 site in the α chain comprise A2BG2S2, FA2BG2S2, F2A2BG2S2 and A3BG3S3;
   and/or,
N-glycans at the Asn69 site in the α chain comprise FA2S2, A2S2, FA3G2S2, A3G2S2, A4G3S3 and A3S3;
   and/or,
N-glycans at the Asn163 site in the α chain comprise FA2G2S2, A2BG2S2, FA2BG2S2, F2A2BG2S2, A3G3S3, FA3G3S3, A3BG3S3and FA3BG3S3;
   and/or,
N-glycans at the Asn183 site in the α chain comprise A2G2S1, A2G2S1M4, A2BG1S1, FA2BG1S1, A2BG2S2, FA2BG2S2 and M5;
   and/or,
N-glycans at the Asn59 site in the β chain comprise A2BG2S2, FA2BG2S2, F2A2BG2S2, A3BG3S3, and further comprise N-glycans containing terminal galactose, preferably, the N-glycans containing terminal galactose comprise A2BG2S1, FA3G3S2, etc.,
   and/or,
N-glycans at the Asn24 site in the γ chain comprise A2BG1S1, FA2BG1S1, FA2BG2S1, A2BG2S2, FA2BG2S2, F2A2BG2S2, further comprise triantennary N-glycan or tetraantennary N-glycan containing fucosyl and/or acetyl sialic acid, preferably the triantennary N-glycan or tetraantennary N-glycan comprise FA3G3S2, FA3BG3S3, A3BG2S2, A3BG3S3, etc..

In some embodiments according to any one of the coagulation factor X activating enzymes described above :
N-glycans at the Asn28 site in the α chain comprise:
A2BG2S2 with a relative content of 22% to 32%, preferably 25% to 30%, more preferably 25%, 25.5%, 26%, 26.5%, 27%, 27.5%, 28%, 28.5%, 29%, 29.5% or 30% ;
FA2BG2S2 with a relative content of 30% to 40%, preferably 34% to 38%, more preferably 34%, 34.5%, 35%, 35.5%, 36%, 36.5%, 37%, 37.5% or 38% ;
F2A2BG2S2 with a relative content of 18% to 28%, preferably 20% to 25%, more preferably 20%, 20.5%, 21%, 21.5%, 22%, 22.5%, 23%, 23.5%, 24%, 24.5% or 25%; and
A3BG3S3 with a relative content of 5% to 10%, preferably 6% to 9%; more preferably 6%, 6.5%, 7%, 7.5%, 8%, 8.5% or 9% ;
   and/or,
N-glycans at the Asn69 site in the α chain comprise:
   FA2S2 with a relative content of 5% to 10%, preferably 6% to 9%, more preferably 6%, 6.5%, 7%, 7.5%, 8%, 8.5% or 9% ;
   A2S2 with a relative content of 5% to 10%, preferably 6% to 8%, more preferably 6%, 6.5%, 7%, 7.5% or 8% ;
   FA3G2S2 with a relative content of 7% to 20%, preferably 9% to 15%, more preferably 9%, 9.5%, 10%, 10.5%, 11%, 11.5%, 12%, 12.5%, 13%, 13.5%, 14%, 14.5% or 15% ;
   A3G2S2 with a relative content of 20% to 30%, preferably 22% to 29%, more preferably 22%, 22.5%, 23%, 23.5%, 24%, 24.5%, 25%, 25.5%, 26%, 26.5%, 27%, 27.5%, 28%, 28.5% or 29% ;
   A4G3S3 with a relative content of 10% to 25%, preferably 15% to 20%, more preferably 15%, 15.5%, 16%, 16.5%, 17%, 17.5%, 18%, 18.5%, 19%, 19.5% or 20%; and
   A3S3 with a relative content of 15% to 30%, preferably 16% to 20%; more preferably 16%, 16.5%, 17%, 17.5%, 18%, 18.5%, 19%, 19.5% or 20% ;
      and/or,
   N-glycans at the Asn163 site in the α chain comprise:
      FA2G2S2 with a relative content of 3% to 8%, preferably 4% to 6%, more preferably 4%, 4.5%, 5%, 5.5% or 6%;
      A2BG2S2 with a relative content of 6% to 12%, preferably 8% to 10%, more preferably 8%, 8.5%, 9%, 9.5% or 10% ;
      FA2BG2S2 with a relative content of 15% to 25%, preferably 19% to 22%, more preferably 19%, 19.5%, 20%, 20.5%, 21%, 21.5% or 22% ;
      F2A2BG2S2 with a relative content of 10% to 25%, preferably 15% to 20%, more preferably 15%, 15.5%, 16%, 16.5%, 17%, 17.5%, 18%, 18.5%, 19%, 19.5% or 20% ;
      A3G3S3 with a relative content of 5% to 10%, preferably 7% to 9%, more preferably 7%, 7.5%, 8%, 8.5% or 9% ;
      FA3G3S3 with a relative content of 5% to 10%, preferably 8% to 9%, more preferably 8%, 8.5% or 9% ;
      A3BG3S3 with a relative content of 5% to 10%, preferably 7% to 9%; more preferably 7%, 7.5%, 8%, 8.5% or 9% and
      FA3BG3S3 with a relative content of 3% to 10%, preferably 5% to 8%; more preferably 5%, 5.5%, 6%, 6.5%, 7%, 7.5% or 8% ;
         and/or,
      N-glycans at the Asn183 site in the α chain comprise:
         A2G2S1 with a relative content of 5% to 15%, preferably 9% to 10%, more preferably 9%, 9.5% or 10%;
         A2G2S1M4 with a relative content of 4% to 8%, preferably 5% to 6%, more preferably 5%, 5.5%, or 6% ;
         A2BG1S1 with a relative content of 10% to 17%, preferably 12% to 14%, more preferably 12%, 12.5%, 13%, 13.5% or 14% ;
         FA2BG1S1 with a relative content of 5% to 10%, preferably 7% to 8%, more preferably 7%, 7.5% or 8%;
         A2BG2S2 with a relative content of 10% to 20%, preferably 14% to 16%, more preferably 14%, 14.5%, 15%, 15.5% or 16% ;
         FA2BG2S2 with a relative content of 6% to 12%, preferably 8% to 10%; more preferably 8%, 8.5%, 9%, 9.5% or 10%; and
         M5 with a relative content of 3% to 8%, preferably 4% to 7%; more preferably 4%, 4.5%, 5%, 5.5%, 6%, 6.5% or 7% ;
            and/or,
         N-glycans at the Asn59 site in the β chain comprise:
            A2BG2S2 with a relative content of 15% to 25%, preferably 19% to 24%, more preferably 19%, 19.5%, 20%, 20.5%, 21%, 21.5%, 22%, 22.5%, 23%, 23.5% or 24% ;
            FA2BG2S2 with a relative content of 30% to 40%, preferably 32% to 34%, more preferably 32%, 32.5%, 33%, 33.5% or 34% ;
            F2A2BG2S2 with a relative content of 20% to 30%, preferably 21% to 27%, more preferably 21%, 21.5%, 22%, 22.5%, 23%, 23.5%, 24%, 24.5%, 25%, 25.5%, 26%, 26.5% or 27% ;
            A3BG3S3 with a relative content of 3% to 10%, preferably 5% to 7%, more preferably 5%, 5.5%, 6%, 6.5% or 7% ;
            N-glycans with terminal galactose with a relative content of 1% to 5%, preferably 2% to 4%, more preferably 2%, 2.5%, 3%, 3.5% or 4% ;
               and/or,
            N-glycans at the Asn24 site in the γ chain comprise:
               A2BG1S1 with a relative content of 4% to 10%, preferably 6% to 8%, more preferably 6%, 6.5%, 7%, 7.5% or 8%;
               FA2BG1S1 with a relative content of 20% to 30%, preferably 24% to 28%, more preferably 24%, 24.5%, 25%, 25.5%, 26%, 26.5%, 27%, 27.5% or 28% ;
               FA2BG2S1 with a relative content of 3% to 8%, preferably 5% to 7%, more preferably 5%, 5.5%, 6%, 6.5% or 7% ;
               A2BG2S2 with a relative content of 9% to 18%, preferably 11% to 14%, more preferably 11%, 11.5%, 12%, 12.5%, 13%, 13.5% or 14% ;
               FA2BG2S2 with a relative content of 20% to 30%, preferably 25% to 28%, more preferably 25%, 25.5%, 26%, 26.5%, 27%, 27.5% or 28% ;
               F2A2BG2S2 with a relative content of 4% to 10%, preferably 6% to 8%, more preferably 6%, 6.5%, 7%, 7.5% or 8%; and
               triantennary N-glycan or tetraantennary N-glycan containing fucosyl and/or acetyl sialic acid with a relative content of 5% to 12%, preferably 6% to 10%; more preferably 6%, 6.5%, 7%, 7.5%, 8%, 8.5%, 9%, 9.5% or 10%.

In some embodiments, the relative content of N-glycans refers to the percentage of the total number of the coagulation factor X activating enzyme molecules containing the particular N-glycans at a certain site to the total number of all coagulation factor X activating enzyme molecules.

In some embodiments the coagulation factor X activating enzyme described above is derived from Russell's viper (Daboia russelii siamensis). In some specific embodiments, the coagulation factor X activating enzyme is isolated from the Russell's viper venom or produced by genetic engineering.

Another aspect of the present application provides a pharmaceutical composition comprising the coagulation factor X activating enzyme described above, and a pharmaceutically acceptable carrier. The pharmaceutical composition of the present application can be obtained by mixing the coagulation factor X activating enzyme having a desired purity with an optional pharmaceutically acceptable carrier, excipient or stabilizer. Further, the pharmaceutical composition of the present application can be prepared in the form of lyophilized formulation or liquid formulation.

The pharmaceutical composition of the present invention may also contain one or more other active compounds as necessary for the particular indication being treated, preferably those with complementary activities that do not adversely affect each other. For example, it may be desirable to further provide other active compounds for stopping bleeding or promote coagulation in addition to the coagulation factor X activating enzyme. Such compounds are suitably present in combination in amounts that are effective for the purpose intended. The effective amount of such other compounds depends on the amount of coagulation factor X activating enzyme in the composition, the type of disease or condition or treatment, and other factors as described in the present application.

Another aspect of the present invention provides a method of stopping bleeding or treatment of the bleeding disorder comprising administering to a subject in need thereof a therapeutically effective amount of the coagulation factor X activating enzyme or a pharmaceutical composition as described above.

Another aspect of the present invention provides the use of the coagulation factor X activating enzyme or a pharmaceutical composition as described above in the manufacture of a medicament for stopping bleeding or treatment of the bleeding disorder.

In some embodiments the bleeding disorder comprises surgical wound bleeding, an internal hemorrhagic disease, a hereditary hemorrhagic disease or a coagulation system disease; In some embodiments the coagulation system disease comprises hemophilia; In some embodiments, the hemophilia is a hemophilia with inhibitors; and in some embodiments, the hemophilia is hemophilia A or hemophilia B.

The coagulation factor X activating enzyme or the pharmaceutical composition of the present application can be administered to an individual (such as a human) via various routes, including, for example, intravenous, intra-arterial, intraperitoneal, intrapulmonary, oral, inhalation, intravascular, intramuscular, intra-tracheal, subcutaneous, intraocular, intrathecal, transmucosal or transdermal.

The coagulation factor X activating enzyme or the pharmaceutical composition to be used for *in vivo* administration must be sterile. This is readily accomplished by, e.g., filtration through sterile filtration membranes.

Another aspect of the present invention provides an article of manufacture and/or kit. Preferably the article of manufacture can comprise a container and a label or package insert on or associated with the container. Suitable containers include, for example, bottles, vials, syringes, etc. The containers may be formed from a variety of materials such as glass or plastic. Generally, the container holds a composition which is effective for treating a disease or disorder described herein, and has a sterile access port (for example, the container may be an intravenous solution bag or a vial having a stopper pierceable by a hypodermic injection needle). At least one of the active substances in the pharmaceutical composition is coagulation factor X activating enzyme described in the present application. The label or package instructions indicate that the composition may be used for stopping bleeding or treatment of the bleeding disorder. Package insert refers to instructions customarily included in commercial packages of therapeutic products that contain information about the indications, usage, dosage, administration, contraindications and/or warnings concerning the use of such therapeutic products. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Another aspect of the present application provides an isolated nucleic acid molecule encoding the coagulation factor X activating enzyme as described above.

Another aspect of the present application provides a vector comprising the nucleic acid molecule as described above. In some embodiments, the vector is a DNA vector. In some embodiments, the vector is a prokaryotic expression vector or a eukaryotic expression vector.

Also, the present application provides a method for inserting the nucleic acid molecule of the present application into a vector. In one embodiment of the present application, the expression of the coagulation factor X activating enzyme by a natural or synthetic nucleic acid encoding the coagulation factor X activating enzyme can be achieved by inserting the nucleic acid into an appropriate expression vector, such that the nucleic acid is operably linked to 5' and 3' regulatory elements, e.g., including a promoter and a 3'untranslated region (UTR), to express the coagulation factor X activating enzyme. The vectors can be suitable for replication and integration in prokaryotic and/or eukaryotic host cells. Typical cloning and expression vectors contain transcription and translation terminators, initiation sequences, and promoters useful for regulation of the expression of the desired nucleic acid sequence. The vector can also comprise one or more selectable marker genes and other genetic elements. One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the application should not be limited to the use of constitutive promoters. Inducible promoters are also contemplated as part of the application. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Another aspect of the present application provides an isolated host cell, wherein the host cell comprises the coagulation factor X activating enzyme, the nucleic acid molecule of the coagulation factor X activating enzyme or the vector as described above. In some embodiments, the host cell is a prokaryotic or eukaryotic cell. In some embodiments, the prokaryotic cell is an E. coli cell.

Methods of introducing nucleic acid molecules or vectors into cells and expressing them are known in the art. Vectors can be readily introduced into host cells by any method known in the art, such as physical, chemical or biological means. Physical methods of introducing a polynucleotide into a host cell include calcium phosphate precipitation, lipofection, particle bombardment, microinjection, electroporation, and the like. Methods for producing cells comprising vectors and/or exogenous nucleic acids are well-known in the art. In some embodiments, the introduction of a polynucleotide into a host cell is carried out by calcium phosphate transfection. Biological methods for introducing a polynucleotide of interest into a host cell include the use of DNA and RNA vectors. Viral vectors, and especially retroviral vectors, have become the most widely used method of inserting genes into mammalian, e.g., human cells. Other viral vectors can be derived from lentivirus, poxviruses, herpes simplex virus 1, adenoviruses and adeno-associated viruses, and the like. Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, *e.g.,* by immunological means (ELISAs and Western blots) falling within the scope of the application.

One aspect of the present application provides a method for the preparation of the coagulation factor activating enzyme as described above, comprising the following steps:
a) Culturing the host cell as described above under conditions that can effectively express the coagulation factor X activating enzyme;
b) Obtaining the expressed coagulation factor X activating enzyme from the host cell.

Another aspect of the present application provides a method for glycosylation modification of the coagulation factor X activating enzyme as described above, and the method is to genetically engineer a host cell to produce the coagulation factor X activating enzyme having a predetermined N-glycosylation modification, and/or treating the coagulation factor X activating enzyme with glycosylation-related enzymes such that the coagulation factor X activating enzyme has a predetermined N-glycosylation modification.

Another aspect of the present application provides a method for the preparation of the coagulation factor X activating enzyme as described above, comprising the following steps:
(a) Obtaining the stock solution of the Russell's viper venom;
(b) Isolating and purifying the stock solution of venom to obtain the coagulation factor activating enzyme as described above.

### The method of preparing and isolating the coagulation factor X activating enzyme

One aspect of the present application provides a method for isolating a coagulation factor X activating enzyme, the method comprises the following steps in turn:
(a) Obtaining a sample of the stock solution of the Russell's viper venom;
(b) Subjecting the sample to anion exchange chromatography;
(c) After anion exchange chromatography purification, subjecting the sample to cation exchange chromatography;
(d) Obtaining the coagulation factor X activating enzyme.

In some preferred embodiments, both anion exchange chromatography and cation exchange chromatography are carried out only once.

In other preferred embodiments, no further purification steps are carried out after cation exchange chromatography, or size exclusion chromatography step is further included after cation exchange chromatography and no further purification steps are carried out.

### Anion exchange chromatography

In some embodiments, the packed medium in the anion exchange chromatography column is agarose or polymer;
Preferably, the chromatography column packed with agarose medium is selected from Sepharose H. P., Sepharose F. F., Capto, or Diamond, more preferably Q Sepharose H. P., Q Sepharose F.F., Capto Q, EDAE-Sepharose FF, Diamond Q or Diamond MIX-A;
Preferably, the packed medium is polymer which is polystyrene/stilbene, polyacrylate, or diethylaminoethyl; preferably, the chromatography column is NenoGel 50 Q, UniGel 65 Q HC or UniGel 30DEAE.

In some embodiments, the elution buffer of the anion exchange chromatography is Tris-HCl buffer containing NaCl with a pH of 8.0 to 8.6, wherein the concentration of Tris-HCl buffer is from 10 mM to 30 mM and the concentration of NaCl is from 0.1M to 0.5M;
Preferably, the pH of Tris-HCl buffer is 8.0, 8.1, 8.2, 8.3, 8.4, 8.5 or 8.6, and the concentration of Tris-HCl buffer is 10mM, 15mM, 20mM, 25mM or 30mM; and the concentration of NaCl is 0.1M, 0.2M, 0.3M, 0.4M or 0.5M;
Preferably, the elution flow rate of the anion exchange column chromatography is from 30 cm/hour to 150 cm/hour, preferably from 80 cm/hour to 120 cm/hour, more preferably 90 cm/hour.

### Cation exchange chromatography

In some embodiments, the packed medium of the cation exchange chromatography column is agarose or polymer;
Preferably, the chromatography column packed with agarose medium is selected from Capto SP ImpRes, CM Beads 6FF or Diamond MMC;
Preferably, the polymer medium is preferably polystyrene/stilbene, and preferably, the chromatography column is NenoGel 50 SP.

In some embodiments, the elution buffer of the cation exchange chromatography is phosphate buffer containing NaCl with a pH of 6.5 to 7.5, wherein the concentration of phosphate buffer is from 10 mM to 30 mM, and the concentration of NaCl is from 0.2M to 0.6M;
Preferably, the pH of phosphate buffer is 6.5, 6.7, 6.8, 6.9, 7.0, 7.2, or 7.5, and the concentration of phosphate buffer is 10mM, 15mM, 20mM, 25mM, or 30mM, and the concentration of NaCl is 0.2 M, 0.3 M, 0.4M, 0.5 M or 0.6 M;
Preferably, the elution flow rate of the cation exchange column chromatography is from 30 cm/hour to 150 cm/hour, preferably 40 cm/hour to 100 cm/hour, more preferably 60 cm/hour.

### Purification step before anion exchange chromatography

In some embodiments, size exclusion chromatography, heparin affinity chromatography, and/or hydrophobic chromatography may further be carried out before anion exchange chromatography.

### Size exclusion chromatography

In some embodiments, the packed medium of the size exclusion chromatography column is selected from gel filtration media, preferably Sephacryl, Superdex or Sephadex, more preferably Sephacryl S-200 HR, Superdex75pg, Superdex200pg or SephadexG25.

Preferably, the elution buffer of the size exclusion chromatography is Tris-HCl buffer with a pH of from 8.0 to 8.6, and the concentration of Tris-HCl buffer is from 10 mM to 30 mM; preferably the pH of Tris-HCl buffer is 8.0, 8.1, 8.2, 8.3, 8.4, 8.5 or 8.6, and the concentration is 10mM, 15mM, 20mM, 25mM or 30mM.

Preferably, the elution flow rate of the size exclusion chromatography is from 30 cm/hour to 150 cm/hour, preferably form 40 cm/hour to 120 cm/hour, more preferably 60 cm/hour.

### Heparin affinity chromatography

In some embodiments, the packed medium of the heparin affinity chromatography column is agarose medium; preferably Capto hips, Heparin Berpharose F.F., Heparin Sepharose 6 F.F., or Heparin Sepharose H.P..

Preferably, the elution buffer of the heparin affinity chromatography is Tris-HCl buffer containing NaCl with a pH of from 7.6 to 8.4, wherein the concentration of Tris-HCl buffer is from 10 mM to 30 mM and the concentration of NaCl is 0.5M to 1.5M.

Preferably, the pH of Tris-HCl buffer is 7.6, 7.8, 8.0, 8.2 or 8.4, and the concentration of Tris-HCl buffer is 10mM, 15mM, 20mM, 25mM or 30mM; and the concentration of NaCl is 0.5 M, 0.7 M, 0.8 M, 0.9 M, 1.0M, 1.1 M, 1.2 M, 1.3 M, 1.4 M or 1.5 M.

Preferably, the elution flow rate of the heparin affinity chromatography is from 30 cm/hour to 150 cm/hour, preferably from 40 cm/hour to 120 cm/hour, more preferably 60 cm/hour.

### Hydrophobic chromatography

In some embodiments, the packed medium of the hydrophobic chromatography column is agarose medium; preferably Butyl Bestarose HP.

Preferably, the elution buffer of the hydrophobic chromatography is phosphate buffer containing NaCl with a pH of from 6.5 to 7.5, wherein the concentration of phosphate buffer is from 10 mM to 30 mM, and the concentration of NaCl is from 0.5M to 2.5M.

Preferably, the pH of the phosphate buffer is 6.5, 6.6, 6.8, 7.0, 7.2 or 7.5, the concentration of phosphate buffer is 10mM, 15mM, 20mM, 25mM or 30mM; and the concentration of NaCl is 0.5M, 1.0M, 1.5 M, 1.7 M, 1.9 M, 2.0M, 2.1 M, 2.2 M, 2.4 M or 2.5 M.

Preferably, the elution flow rate of the hydrophobic chromatography is from 30 cm/hour to 150 cm/hour, preferably from 40 cm/hour to 120 cm/hour, more preferably 60 cm/hour.

### Size exclusion chromatography after cation exchange chromatography

In some embodiments, there is further comprising size exclusion chromatography after the cation exchange chromatography;
Preferably, the packed medium of the size exclusion chromatography is gel filtration media, preferably Sephacryl, Superdex or Sephadex, more preferably Sephacryl S-200 HR, Superdex75pg, Superdex200pg or SephadexG25.

Preferably, the elution buffer of the size exclusion chromatography is phosphate buffer with a pH of from 6.5 to 7.5, and the concentration of phosphate buffer is from 40 mM to 80 mM, more preferably the pH of phosphate buffer is 6.5, 6.6, 6.8, 7.0, 7.2 or 7.5, and the concentration of phosphate buffer is 40mM, 50mM, 60mM, 70mM or 80mM;
Preferably, the elution flow rate of the size exclusion chromatography is from 5 cm/hour to 80 cm/hour, preferably from 10 cm/hour to 35 cm/hour, more preferably 20 cm/hour.

### Ultrafiltration

In some embodiments, there is further comprising ultrafiltration after anion exchange chromatography and before cation exchange chromatography;
Preferably, the specific steps of ultrafiltration are: collecting the components corresponding to the target peak of anion exchange chromatography and concentrating 5-20 times with an ultrafiltration membrane, and then exchanging the buffer of the components corresponding to the target peak with 10 mM-30 mM phosphate buffer, wherein the pH of the phosphate buffer is from 6.5 to 7.5, and the molecular weight cut-off of ultrafiltration membrane is form 10kD to 50kD;
More preferably, the concentration of phosphate buffer is 10mM, 15mM, 20mM, 25mM or 30mM, the pH of phosphate buffer is 6.5, 6.6, 6.8, 7.0, 7.2 or 7.5, the molecular weight cut-off of ultrafiltration membrane is 30kD, and the concentration ratio is 10 times.

### Viral inactivation

In some embodiments, after obtaining a sample of the stock solution of snake venom and before anion chromatography purification, there is further comprising the step of treating the stock solution of the snake venom with viral inactivation; preferably the viral inactivation treatment is carried out by the S/D (solvent/detergent) method.

In some preferred embodiments, the isolation methods described above can be used to isolate or prepare the coagulation factor X activating enzyme described in the present application.

### Glycosylation analysis method

The present application also provides a method of analyzing the glycosylation of the coagulation factor X activating enzyme, and the method comprises the followed steps:
(1) treating the coagulation factor X activating enzyme samples.
(2) performing enzymolysis on the treated sample.
(3) analyzing the enzymatically hydrolyzed sample to obtain the glycan profile or glycosylation status of the coagulation factor X activating enzyme.

In a specific embodiment, the step of treating the coagulation factor X activating enzyme sample in step (1) comprises urea solution treatment, DTT treatment and iodoacetamide alkylation treatment. In some preferred embodiments, step (1) is: adding urea solution to the coagulation factor X activating enzyme sample, centrifuging, and sequentially performing reduction with DTT (dithiothreitol) and alkylation with iodoacetamide.

In some embodiments, the concentration of urea is from 4M to 10M, preferably 8M.

In some embodiments, the concentration of DTT is from 5mM to 40mM, preferably 10mM.

In some embodiments, the DTT reduction condition is from 45 °C to 65 °C, preferably 56 °C.

In some embodiments, the concentration of iodoacetamide is from 20mM to 50mM, preferably 30mM.

In another specific embodiment, the enzymatic step in step (2) comprises the following steps: adding ammonium bicarbonate, followed by adding trypsin for treatment, and terminating the reaction by adding formic acid after the enzymatic hydrolysis reaction. In some preferred embodiments, the step (2) is: adding ammonium bicarbonate solution, centrifugation, followed by adding trypsin for enzymatic hydrolysis, and terminating the reaction by adding formic acid solution after the enzymatic reaction, and performing ultrafiltration on the sample.

In some embodiments, the concentration of ammonium bicarbonate is from 30mM to 80 mM, preferably 50mM, preferably, the step of adding ammonium bicarbonate is repeated 2 times;
In some embodiments, the ratio of trypsin is from 1: 30 to 1: 80, preferably 1: 50, preferably, the step of adding trypsin is repeated 2 times, and the reaction is performed at 37 °C.

In a specific embodiment, the analysis is performed by liquid chromatography-mass spectrometry in step (3).

In some embodiments, the mobile phase of liquid chromatography contains formic acid. In some preferred embodiments, formic acid is added in a ratio of from 1:5 to 1: 30, preferably 1: 20.

In some embodiments, the column for liquid chromatography is a C18 column;
In some embodiments, the column temperature of the chromatography column is from 35 °C to 55 °C, preferably from 35 °C to 45 °C, more preferably 40 °C;
In some embodiments, the flow rate of the liquid chromatography is from 0.1 ml/min to 1 ml/min, preferably from 0.1 ml/min to 0.8 ml/min, more preferably 0.2 ml/min;
In some embodiments, the loading of the liquid chromatography is from 2µl to 20µl, preferably from 4 to 15µl, more preferably from 6µl to 10µl;
In some embodiments, the mobile phase of liquid chromatography consists of mobile phase A and mobile phase B, wherein mobile phase A is an aqueous solution of formic acid and trifluoroacetic acid, preferably an aqueous solution of (0.01% -0.2%) formic acid+ (0.01% -0.2%) trifluoroacetic acid, more preferably an aqueous solution of (0.05% -0.1%) formic acid+ (0.05% -0.1%) trifluoroacetic acid;
Mobile phase B is an acetonitrile aqueous solution of formic acid and trifluoroacetic acid, preferably an acetonitrile solution of (0.01% -0.2%) formic acid+ (0.01% -0.2%) trifluoroacetic acid, more preferably an acetonitrile solution of (0.05% -0.1%) formic acid+ (0.05% -0.1%) trifluoroacetic acid, preferably the acetonitrile solution contains (80% -100%) acetonitrile and (0-20%) water, preferably 90% acetonitrile and 10% water.

In some preferred embodiments, the glycosylation analysis described above can be used to analyze the coagulation factor X activating enzyme described in the present application.

### Terms and definitions

In order to facilitate understanding of the contents and embodiments of the present application, the meanings of specific terms are as follows.

Glycosylation: A process of modifying the protein by additional sugar chains. There are usually two types of glycosylations, O-linked glycosylation and N-linked glycosylation, respectively. O-linked glycosylation refers the addition of glycans to the hydroxyl groups, such as serine (Ser) and/or threonine (Thr) residue of a protein molecule. N-linked glycosylation refers to the addition of glycans to amide groups, such as asparagine acid (Asn) residue of a protein molecule. Proteins are glycosylated to form glycoproteins.

Glycans: refer to polysaccharides, oligosaccharides or monosaccharides. Glycans may be monomers or polymers of sugar residues, or may be linear or branched. Glycans may include natural sugar residues (e.g., glucose, N-acetylglucosamine, N-acetylneuraminic acid, galactose, mannose, fucose, hexose, arabinose, ribose, xylose, etc.) and/or modified sugars (e.g., 2 '-fluororibose, 2' -deoxyribose, mannose phosphate, 6 '-sulfo N-acetylglucosamine, etc.). Glycans may comprise homopolymers and heteropolymers of sugar residues. Glycans also encompass a glycan component of a glycoconjugate (e. g., a glycan component of a glycoprotein, glycolipid, proteoglycan). Glycans also encompass free glycans, including a glycan that that has been cleaved by a glycoconjugate or otherwise released.

N-glycans: refer to N-linked polysaccharides or oligosaccharides. N-linked polysaccharides or oligosaccharides refers to, for example, a polysaccharide or oligosaccharide linked to an amide group of asparagine or arginine residues in a protein by N-acetylglucosamine residues, the basic structure, composition and type of which are known in the art (see, e. g., Drickamer K, Taylor ME (2006), Introduction to Glycobiology, Vol. 2nd ed. Editor: Ajit Varki et.al., Essentials of Glycobiology, 4th edition, Cold Spring Harbor (NY): Cold Spring Harbor Laboratory Press, 2022, etc.). In particular, the major sugars that make up N-glycans include glucose, galactose, mannose, fucose, N-acetylgalactosamine (GalNAc), N-acetylglucosamine (GlcNAc), sialic acid (SA), etc. In some embodiments, the core structure of the N-glycan is known in the art, i. e., each has a pentasaccharide core ( 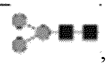 wherein 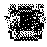 represents acetylglucosamine GlcNAc; 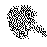 represents mannose Man and can also be represented as Man₃GlcNAc₂). N-glycans extend from the basic pentasaccharide core. According to the branched chain components of the N-glycan, N-glycans can be classified, including high mannose type, complex type, or hybrid type. The "high mannose" N-glycans have 5 or more mannose residues. "Complex" N-glycans typically have at least one GlcNAc linked to the 1,3 mannose arm of the pentasaccharide core. Complex N-glycans may also have galactose or N-acetylgalactosamine residues, which may be modified with sialic acid or derivatives. Complex N-glycans may also have multiple antennas on the pentasaccharide core, often referred to as "multi-antenna glycans". The "hybrid" N-glycans have at least one GlcNAc at the end of the 1 '3 mannose arm of the pentasaccharide core and 0 or more mannose at the 1' 6 mannose arm of the pentasaccharide core.

The nomenclature rules for glycotypes are also well known to those skilled in the art, and can be named, for example, using the Oxford Notation method (see, e. g., David J. Harvey et al., Proposal for a standard system for drawing systems of N-and O-linked asset and related compounds, Proteomics 2009,9,3796-3801, Margaret Doherty et al., Plasma N-glycans in colorectal cancer risk, SCIENTIFIC REPORTS (2018) 8: 8655, Jerrard M Hayes et al. Identification of Fc Gamma Receptor Glycoforms That Produce Differential Binding Kinetics for Rituximab, Mol Cell Proteomics 2017Oct, 16 (10): 1770-1788, https://www.ludger.com/product-catalogue/standards-controls/n-glycan- unlabelled, etc.). For example, taking F2A3BG3S3 as an example, wherein F represents fucose, the number after F represents its number, F2 represents that there are 2 fucoses, A represents antennary, i. e., adding acetylglucosamine (GlcNAc) to the mannose(Man) of the pentasaccharide core, and the number after A represents the number of its antennary, that is, A3 represents that its sugar chain is three antennas; B represents bisecting GlcNAc, i. e. a acetylglucosamine(GlcNAc) is attached to the mannose (Man) in the middle of the pentasaccharide core, G represents galactose, and the number following it represents its number, G3 represents that there are 3 galactoses, S represents sialic acid, and the number following it represents its number, S3 represents that there are 3 sialic acid.

N-glycosylation: "N-glycosylation" and "N-linked glycosylation" are used interchangeably and refer to a process in which a sugar is linked to a linking group comprising an asparagine residue or an arginine residue, or comprising an N-linked glycosylation site or motif.

Isolated: "Isolated" biological components (e.g., nucleic acid molecules, proteins, viruses, or cells) have been substantially isolated or purified from cells or tissues of organisms in which the components naturally occur or other biological components (e.g., other chromosomal and extrachromosomal DNA and RNA, proteins and cells) in the organism itself. Nucleic acid molecules and proteins that have been "isolated" include those purified by standard purification methods. The term also includes nucleic acid molecules and proteins prepared by recombinant expression in a host cell, as well as chemically synthesized nucleic acid molecules and proteins.

Pharmaceutically acceptable carriers: include any standard pharmaceutical carrier, such as phosphate buffer aqueous solutions, emulsions (e.g., oil/water or water/oil emulsions), and various types of humectants.

Treatment: a method of obtaining beneficial or desired results, including clinical results. For purposes of the present application, the beneficial or desired clinical results include, but are not limited to, one or more of the following: alleviating one or more symptoms resulting from the disease, diminishing the extent of the disease, stabilizing the disease (e. g., preventing or delaying the worsening of the disease), preventing or delaying the recurrence of the disease, delaying or slowing the progression of the disease, ameliorating the disease state, providing a remission (partial or total) of the disease, decreasing the dose of one or more other medications to treat the disease, delaying the progression of the disease, improving or enhancing the quality of life, and/or prolonging the life span.

The therapeutically effective amount: an amount of a particular drug or therapeutic agent (e. g., coagulation factor X activating enzyme) sufficient to achieve a desired effect in a subject or cell treated with the agent. The effective amount of the agent depends on a variety of factors, including, but not limited to, the subject or cell to be treated, and the mode of administration of the therapeutic composition.

Signal peptide: refers to a propeptide that exists as an N-terminal peptide in the form of a protein precursor. The function of the signal peptide is to promote translocation of the expressed polypeptide linked to the endoplasmic reticulum, the signal peptide is usually cleaved by the signal peptidase during this process. The signal peptide may be heterologous or homologous to the organism producing the polypeptide.

The beneficial effects of the present application are as follows:
1. The present application provides a novel Russell's viper coagulation factor X activating enzyme, which has specific N-glycosylation modifications at the 4 sites of Asn 28, Asn 69, Asn 163, Asn 183 in the α chain, Asn 59 site in the β chain, and Asn 24 site in the γ chain. This coagulation factor X activating enzyme has a higher activity than other RVV-X in the prior art and will play an important role in the treatment of clinical hemorrhages and hemorrhagic diseases.
2. The present application also provides a novel method for isolating coagulation factor X activating enzyme from the venom of Russell's viper. Compared with the method in the prior art, the method has a higher yield while ensuring the high purity and high activity of the product, and greatly reduces the production cost, so it is more suitable for large-scale production.

Of course, it should be noted that the method of the embodiments of the present application need not achieve all the above advantages simultaneously.

### BRIEF DESCRIPTION OF DRAWINGS

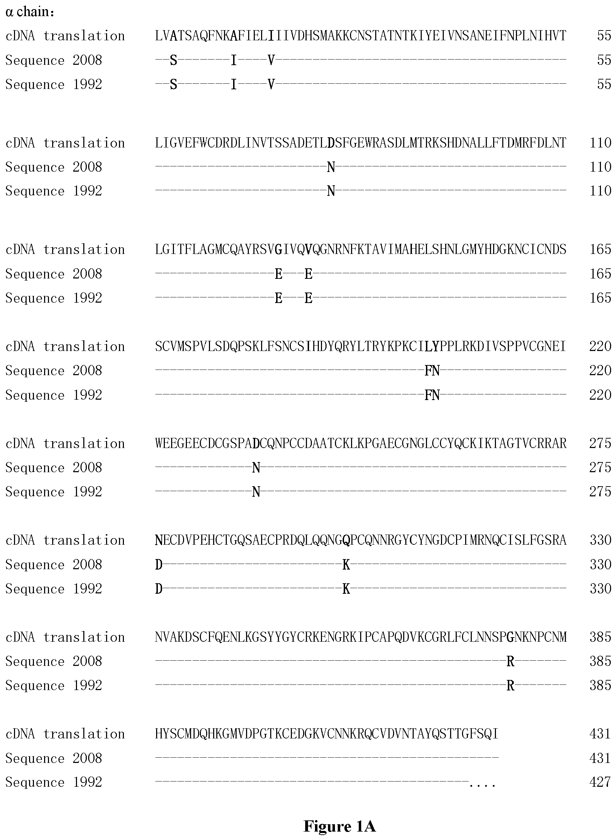

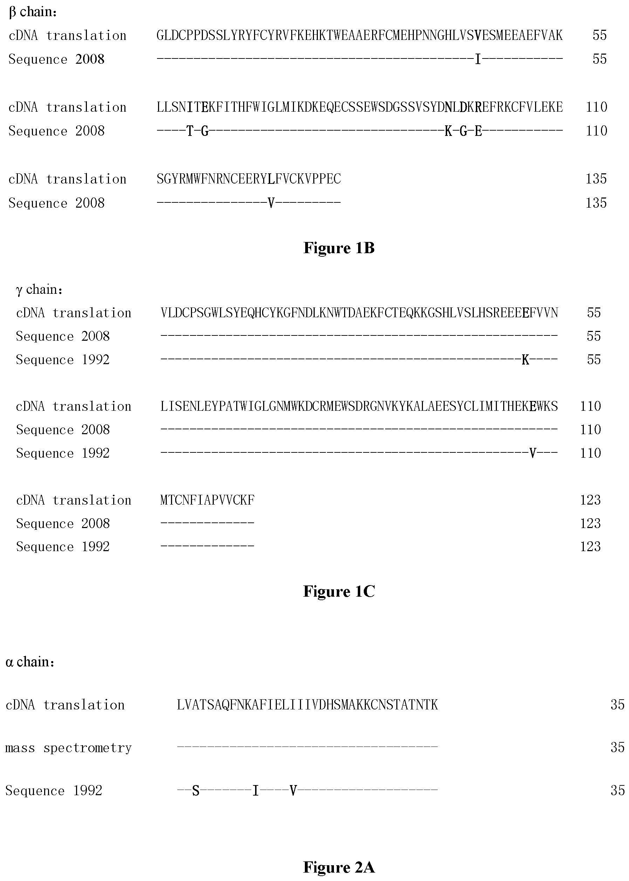

Figures 1A-1C show the comparison results of the α chain cDNA translation sequence of the coagulation factor X activating enzyme with sequence 2008 and sequence 1992 (Figure 1A), the β chain cDNA translation sequence with sequence 2008 (Figure 1B), and the γ chain cDNA translation sequence with sequence 2008 and sequence 1992 (Figure 1C), respectively.

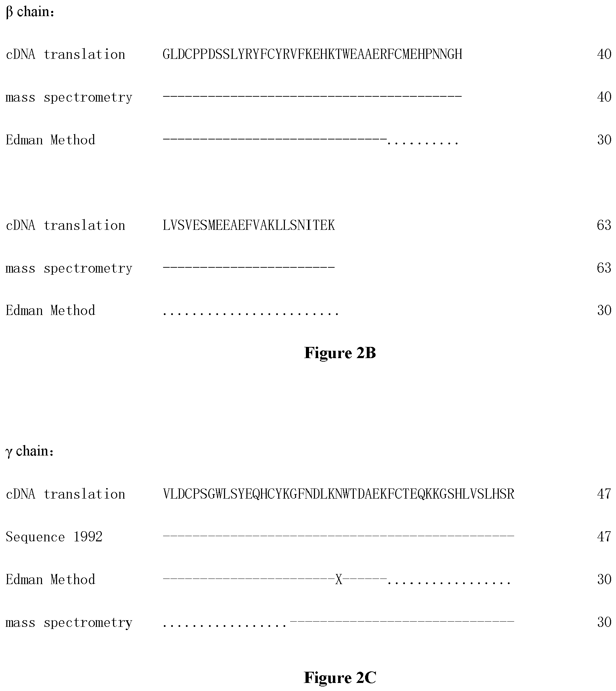

Figures 2A-2C show the determination and comparison results of the N-terminal sequences of the α chain (Figure 2A), β chain (Figure 2B) and γ chain (Figure 2C) of the coagulation factor X activating enzyme.

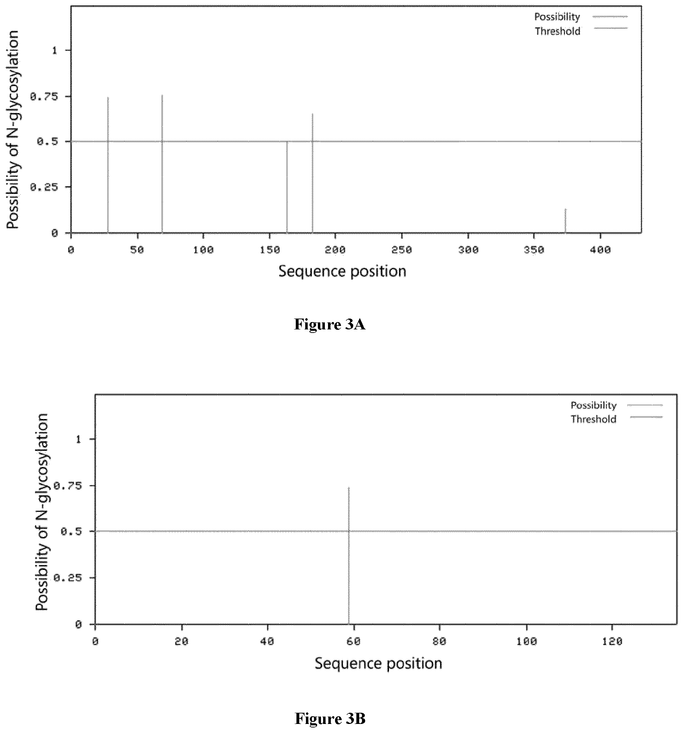

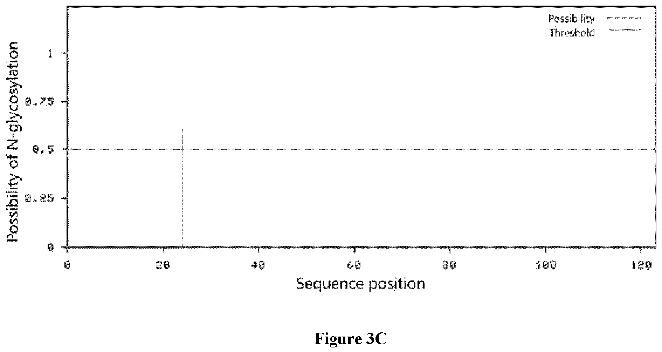

Figures. 3A-3C show the prediction results of N-glycosylation sites of the α chain (Figure. 3A), β chain (Figure. 3B) and γ chain (Figure. 3C) of the coagulation factor X activating enzyme, respectively, wherein the vertical line indicates the possibility of N-glycosylation, the corresponding horizontal coordinate indicates the possible N-glycosylation sites, and the horizontal line indicates the threshold for the possibility of N-glycosylation.

### EXAMPLES

### Example 1. Designing the specific amplification primers of the cDNA of coagulation factor X activating enzyme

The homologous conserved sequence of each chain was analyzed using the Align X function in Vector NTI 11.5 software according to the sequence of the known coagulation factor X activating enzyme three chains (i.e., the α, β and γ chains). According to the analysis results, specific primers for cloning the cDNA coding sequence of three chains were designed, and primers were synthesized by Invitrogen. The sequences of primers were shown in Table 1.

**Tablet Amplification primers of cDNA of the coagulation factor X activating enzyme three chains**

| **Primer name** | **Sequence (5'-3')** |
|---|---|
| αPF | (SEQ ID NO: 1) ATGATGCAAGTTCTCTTAGTAACTATAAGCT |
| αPR | (SEQ ID NO: 2) TCAAATCTGAGAGAAGCCAGTGGTTG |
| βPF | (SEQ ID NO: 3) ATGGGGCGATTCATCTCCGTCAGCTT |
| βPR | (SEQ ID NO: 4) GGATCTTAACACTCTGGTGGCACCTTGCAG |
| γPF | (SEQ ID NO: 5) AGGAAGGAAGACCATGGGGCGATTCATC |
| γPR | (SEQ ID NO: 6) CCTAGAACTTGCACACGACAGGTGCTAT |

### Example 2. Amplification of the cDNA sequence of the coagulation factor X activating enzyme

### 1. Extraction of total RNA and reverse transcription

The head of Russell's viper was cut and quickly frozen with dry ice, and both sides of the venom glands were separated under freezing conditions, crushed and added to a centrifuge tube containing 1 ml Trizol (Takara,# 9108-1), the tissue was blown repeatedly until the tissue was lysis and left for 5 min at room temperature, then 200µl chloroform was added, vortexed and mixed evenly, left for 5 min at room temperature, and then centrifuged at 12000 rpm for 10 min. The supernatant was carefully aspirated into another centrifuge tube, an equal volume of isopropanol was added, mixed well and left at room temperature for 10 min, and centrifuged at 12000 rpm for 10 min. The supernatant was discarded, 1 ml of 75% ethanol was added to the centrifuge tube, and centrifuged at 12000 rpm for 10 min, the supernatant was removed as much as possible, air dried for 5 min, RNA was dissolved by addition of 20µl DEPC water and stored frozen.

The extracted total RNA was subjected to reverse transcription using a reverse transcription kit (Fermentas, # K1622), the reaction solution was prepared according to the instructions, 4µl of total RNA was aspirated, 7µl of DEPC water and 1µl of Oligo T were added and mixed, incubated at 65 °C for 5 min, and immediately subjected to ice bath. 4µl 5× buffer, 1 µl Rnase Inhibitor, 1 µl MMLV and 2 µl dNTPs were added to the reaction, and centrifuged slightly after mixed, incubated at 42 °C for 60 min, and treated at 75 °C for 5 min. The cDNA product obtained can be used directly for PCR amplification.

### 2. Specific amplification of reverse transcribed cDNA

The cDNA obtained by reverse transcription was used as a template, PCR amplification was performed using three pairs of primers synthesized in Example 1, respectively. The reaction systems were shown in Table 2.

**Table 2 The cDNA amplification systems of the coagulation factor X activating enzyme**

| **reagent** | **α chain** | **β chain** | **γ chain** |
|---|---|---|---|
| 10 × PCR Buffer | 5 µl | 5 µl | 5 µl |
| dNTPs (2mM) | 5 µl | 5 µl | 5 µl |
| Upstream primers (5µM) | αPF 5µl | βPF 5µl | γPF 5 µl |
| Downstream primers (5µM) | αPR 5 µl | βPR 5µl | γPR 5 µl |
| cDNA template | 2µl | 2µl | 2µl |
| Taq enzyme (5U/µl) | 0.5µl | 0.5µl | 0.5µl |
| H₂O | 27.5µl | 27.5µl | 27.5µl |

### PCR reaction conditions were as follows:

Degeneration at 94°C for 3min
Degeneration at 94°C for 1min
Annealing at 58°C for 1min The temperature is reduced by 1°C per cycle ×4 cycles
Extension at 72°C for 1min
Degeneration at 94°C for 1min
Annealing at 55°C for 1min ×28 cycles
Extension at 72°C for 1min
Extension at 72°C for 10min

The PCR product was analyzed by agarose gel electrophoresis, and the amplified bands were single, clear and in the same size as expected. The bands were cut into EP tubes and stored at 4°C.

### 3. Gel recovery, ligation of T vector and sequencing

The target bands were recovered by using a gel recovery kit (TIANGEN #DP209-02), the recovered products were ligated into T vector, transformed into E.coli competent cells, and expanded in plate. Five monoclones were selected into LB liquid medium for expansion, and the plasmid was extracted and sent to Takara Company for nucleic acid sequencing. The cDNA sequences of α chain, β chain and γ chain of the coagulation factor X activating enzyme were obtained, respectively.

### Example 3. Determination of the amino acid sequence of the coagulation factor X activating enzyme.

### 1. Analysis of α chain

The open reading frame (ORF) analysis was performed by using Vector NTI based on the cDNA sequence information of the α chain obtained in Example 2. According to the analysis results, the translated protein sequences are as follows (SEQ ID NO: 7), wherein the amino acids at positions 1-20 are signal peptide sequences (shown in bold), the amino acids at positions 21-188 are propeptide sequences (shown with underlines), and the amino acids at positions 189-619 are mature peptide sequences.

10 20 30 40 50
MMQVLLVTIS LAVFPYQGSS IILESGNVND YEVVYPQKVT ALPKGAVQQP
60 70 80 90 100
EQKYEDTMQY EFEVNGEPVV LHLEKNKILF SEDYSETHYS PDGREITTNP
110 120 130 140 150
PVEDHCYYHG RIQNDAHSSA SISACNGLKG HFKLRGEMYF IEPLKLSNNE
160 170 180 190 200
AHAVYKYENI EKEDETPKMC GVTQTNWESD EPIKKASQLV ATSAQFNKAF
210 220 230 240 250
IELIIIVDHS MAKKCNSTAT NTKIYEIVNS ANEIFNPLNI HVTLIGVEFW
260 270 280 290 300
CDRDLINVTS SADETLDSFG EWRASDLMTR KSHDNALLFT DMRFDLNTLG
310 320 330 340 350
ITFLAGMCQA YRSVGIVQVQ GNRNFKTAVI MAHELSHNLG MYHDGKNCIC
360 370 380 390 400
NDSSCVMSPV LSDQPSKLFS NCSIHDYQRY LTRYKPKCIL YPPLRKDIVS
410 420 430 440 450
PPVCGNEIWE EGEECDCGSP ADCQNPCCDA ATCKLKPGAE CGNGLCCYQC
460 470 480 490 500
KIKTAGTVCR RARNECDVPE HCTGQSAECP RDQLQQNGQP CQNNRGYCYN
510 520 530 540 550
GDCPIMRNQC ISLFGSRANV AKDSCFQENL KGSYYGYCRK ENGRKIPCAP
560 570 580 590 600
QDVKCGRLFC LNNSPGNKNP CNMHYSCMDQ HKGMVDPGTK CEDGKVCNNK
610

RQCVDVNTAY QSTTGFSQI

The amino acid sequence of the α chain without the signal peptide and the propeptide is as follows (SEQ ID NO: 8, 431 amino acids):

10 20 30 40 50

LVATSAQFNK AFIELIIIVD HSMAKKCNST ATNTKIYEIV NSANEIFNPL

60 70 80 90 100

NIHVTLIGVE FWCDRDLINVT SSADETLDS FGEWRASDLM TRKSHDNALL

110 120 130 140 150

FTDMRFDLNT LGITFLAGMC QAYRSVGIVQ VQGNRNFKTA VIMAHELSHN

160 170 180 190 200

LGMYHDGKNC ICNDSSCVMS PVLSDQPSKL FSNCSIHDYQ RYLTRYKPKC

210 220 230 240 250

ILYPPLRKDI VSPPVCGNEI WEEGEECDCG SPADCQNPCC DAATCKLKPG

260 270 280 290 300

AECGNGLCCY QCKIKTAGTV CRRARNECDV PEHCTGQSAE CPRDQLQQNG

310 320 330 340 350

QPCQNNRGYC YNGDCPIMRN QCISLFGSRA NVAKDSCFQE NLKGSYYGYC

360 370 380 390 400

RKENGRKIPC APQDVKCGRL FCLNNSPGNK NPCNMHYSCM DQHKGMVDPG

410 420 430

TKCEDGKVCN NKRQCVDVNT AYQSTTGFSQ I

### 2. Analysis of β chain

The open reading frame (ORF) analysis was performed by using Vector NTI based on the cDNA sequence information of the β chain obtained in Example 2. According to the analysis results, the translated protein sequences are as follows (SEQ ID NO: 9), wherein the amino acids at positions 1-23 are signal peptide sequences (shown in bold), and the amino acids at positions 24-158 are mature peptide sequences.

10 20 30 40 50
MGRFISVSFG LLVVFLSLSG TGAGLDCPPD SSLYRYFCYR VFKEHKTWEA
60 70 80 90 100
AERFCMEHPN NGHLVSVESM EEAEFVAKLL SNITEKFITH FWIGLMIKDK
110 120 130 140 150
EQECSSEWSD GSSVSYDNLD KREFRKCFVL EKESGYRMWF NRNCEERYLF
158

VCKVPPEC

The amino acid sequence of the β chain without the signal peptide is as follows (SEQ ID NO: 10, 135 amino acids):

10 20 30 40 50
GLDCPPDSSL YRYFCYRVFK EHKTWEAAER FCMEHPNNGH LVSVESMEEA
60 70 80 90 100
EFVAKLLSNI TEKFITHFWI GLMIKDKEQE CSSEWSDGSS VSYDNLDKRE
110 120 130
FRKCFVLEKE SGYRMWFNRN CEERYLFVCK VPPEC

### 3. Analysis of γ chain

The open reading frame (ORF) analysis was performed using Vector NTI based on the cDNA sequence information of the β chain obtained in Example 2. According to the analysis results, the translated protein sequences are as follows (SEQ ID NO: 11), wherein the amino acids at positions 1-23 are signal peptide sequences (shown in bold), and the amino acids at positions 24-146 are mature peptide sequences.

10 20 30 40 50
MGRFISVSFG CLVVFLSLSG TEAVLDCPSG WLSYEQHCYK GFNDLKNWTD
60 70 80 90 100
AEKFCTEQKK GSHLVSLHSR EEEEFVVNLI SENLEYPATW IGLGNMWKDC
110 120 130 140
RMEWSDRGNV KYKALAEESY CLIMITHEKE WKSMTCNFIA PVVCKF

The amino acid sequence of the γ chain without the signal peptide is as follows (SEQ ID NO: 12, 123 amino acids):

10 20 30 40 50
VLDCPSGWLS YEQHCYKGFN DLKNWTDAEK FCTEQKKGSH LVSLHSREEE
60 70 80 90 100
EFVVNLISEN LEYPATWIGL GNMWKDCRME WSDRGNVKYK ALAEESYCLI
110 120
MITHEKEWKS MTCNFIAPVV CKF

### Example 4. Structural verification of the coagulation factor X activating enzyme amino acid sequence.

### 1. Alignment of amino acid sequences

The amino acid sequences of three mature peptides (i.e., the α, β and γ chains, represented by "cDNA translation") obtained in Example 3 were respectively compared with the amino acid sequence (represented by "sequence 2008") translated from the cDNA sequence (Genbank accession number is DQ137799, AY734997, AY734998, respectively) measured by Chen H.S in 2008 (Chen HS et al., New insights into the functions and N-glycan structures of factor X activating enzyme from Russell's viper venom. FEBS J. 2008 Aug;275(15):3944-58), and the amino acid sequence (Only α and γ chain sequences were detected, indicated by "sequence 1992") measured by Takeya H. by using the Edman degradation method in 1992(Takeya H et al., Coagulation factor X activating enzyme from Russell's viper venom (RW-X). A novel metalloproteinase with disintegrin (platelet aggregation inhibitor)-like and C-type lectin-like domains. J Biol Chem. 1992 Jul 15;267(20):14109-17.), the alignment results were shown in Figures. 1A-1C, wherein the bold letters indicate that the amino acid residues at that position are different, the horizontal line "-" indicates that the amino acid residues at that position are identical, and the point "." indicates that the amino acid residues at that position are not detected.

The comparison results of the α chain cDNA translation sequence with sequence 2008 and sequence 1992 was shown in Figure 1A. The comparison results of the β chain cDNA translation sequence with sequence 2008 was shown in Figure 1B. The comparison results of the γ chain cDNA translation sequence with sequence2008 and sequence 1992 was shown in Figure 1C.

### 2. Analysis and confirmation of N-terminal sequence

In order to confirm the existence of the mature peptide obtained by the above-mentioned cDNA translation in Russell's viper, the coagulation factor X activating enzyme was further extracted, purified and obtained from the venom of Russell's viper. The N-terminal sequences of the α chain, β chain and γ chain of the obtained coagulation factor X activating enzyme were determined by tandem mass spectrometry and Edamn degradation method by Beijing Proteome Research Center and College of Life Science of Peking University, respectively.

The amino acid sequence of the mature peptide obtained from α chain, β chain and γ chain cDNA sequence translation (represented by "cDNA translation"), the sequence identification results by mass spectrometry (represented by "mass spectrometry"), and the determination results by Edman degradation method (represented by "Edman method") were compared with the "sequence 1992", and the results were shown in Figures 2A-2C.

The sequence determination and comparison results of the N-terminal of the α chain were shown in Figure 2A, the 35 amino acids at the N-terminal of the α chain identified by mass spectrometry were consistent with the amino acid sequences at the same position of the cDNA translation sequence.

The analysis of the amino acid sequence at the N-terminal of the α chain by Edman degradation method showed that the detection result of each position was not a single amino acid, and the complete sequence could not be obtained. This was consistent with Kisiel W et al. 's observation of the non-uniformity of the N-terminal amino acids of RVV-X α chain by Edman degradation method in 1976 (Kisiel W et al., Factor X activating enzyme from Russell's viper venom: isolation and characterization. Biochemistry. 1976 Nov 2;15(22):4901-6.).

The sequence determination and comparison results of the N-terminal of the β chain were shown in Figure 2B, the 63 amino acids at the N-terminal of the β chain identified by mass spectrometry were consistent with the amino acid sequence at the same position of the cDNA translation sequence, and the 30 amino acids at the N-terminal determined by Edman degradation method were consistent with the amino acid sequence at the same position of the cDNA translation sequence.

The sequence determination and comparison results of the N-terminal of the γ chain were shown in Figure 2C, wherein "X" represents the undetermined amino acid. A total of 30 amino acids at the N-terminal were identified by Edman degradation. Except the 24th amino acid was not captured, the rest amino acids were consistent with the cDNA translation sequence and sequence 1992. The first 17 amino acids at the N-terminal of γ chain were not captured by mass spectrometry, and the amino acid sequences at positions 18 to 47 were consistent with the amino acid sequence at the same position of the cDNA translation sequence and sequence 1992.

According to the results of the subsequent glycosylation site confirmation test, the amino acid at position 24 is N (Asn, asparagine), and there is N-glycosylation modification. It is speculated that the reason for not capturing the 24th amino acid residue may be that the glycosylation modification affects the determination of this amino acid by Edman degradation method.

In summary, the N-terminal amino acid sequence of the three chains of the coagulation factor X activating enzyme described herein is:
35 amino acid residues at the N-terminal of the α chain (SEQ ID NO: 13): LVATSAQFNKAFIELIIIVDHSMAKKCNSTATNTK
63 amino acid residues at the N-terminal of the β chain (SEQ ID NO: 14): 
GLDCPPDSSLYRYFCYRVFKEHKTWEAAERFCMEHPNNGHLVSVESMEEAEFVAK
LLSNITEK
47 amino acid residues at the N-terminal of the γ chain (SEQ ID NO: 15): VLDCPSGWLSYEQHCYKGFNDLKNWTDAEKFCTEQKKGSHLVSLHSR

The N-terminal amino acid sequencing result of the coagulation factor X activating enzyme was consistent with the N-terminal sequence of the mature peptide obtained from the cDNA translation, indicating that the mature peptide obtained by cDNA translation exists in the venom of Russell's viper.

Through the above structural confirmation and comparison with the sequences in GeneBank and EMBL library, it is confirmed that the present application obtains a new coagulation factor X activating enzyme from the Russell's viper.

### Example 5. Confirmation of the glycosylation site of the coagulation factor X activating enzyme

The cDNA translation sequences of α, β and γ chain of the coagulation factor X activating enzyme were analyzed to predict the possible N-glycosylation sites of these three chains by using NeNGlycl.0Server neural network software (website: http://www.cbs.dtu.dk/ services/NetNGlyc/).

The analysis results of α chain, β chain and γ chain were shown in Figure 3A-3C. The position corresponding to the vertical line in the figure indicates that the site has the sequence feature of N-glycosylation modification, i.e. Asn-X-Thr/Ser. The vertical line indicates the possibility of N-glycosylation. The horizontal line indicates the threshold for the possibility of N-glycosylation, and the threshold is 0.5. If the score value≥0.5, this site is considered to be the predicted glycosylation site.

The prediction results of N-glycosylation sites of the α chain were shown in Figure 3A. The possible N-glycosylation sites from the N-terminus were 28NSTA, 69NVTS, and 183NCSI, and the score values were 0.7407, 0.7516, and 0.6519, respectively. The score value of 163NDSS was 0.4979, close to 0.5, which is also subjected to subsequent experimental analysis as a potential site for N-glycosylation modification.

The predicted results of N-glycosylation sites of the β-chain were shown in Figure 3B. The possible N-glycosylation site was 59NITE, and the score value was 0.7391.

The prediction results of N-glycosylation sites of the γ-chain were shown in Figure 3C. The possible N-glycosylation site was 24NWTD, and the score was 0.6089.

Subsequently, the Beijing Protein Group Research Centre was commissioned to analyze and confirm the 6 N-glycosylation sites of these three chains by mass spectrometry. The results showed that N-glycosylation modification occurred at all 6 sites, which was consistent with the predicted results.

### Example 6. The novel preparation method of the coagulation factor X activating enzyme

### 1. Extraction of the coagulation factor X activating enzyme

The example explored the process of extracting the coagulation factor X activating enzyme from the Russell's viper venom.

Approximately 10 ml of the Russell's viper venom was taken, diluted with 20mMTris-HCl solution (pH 8.5) to a protein concentration of approximately 20-30 mg/ml, centrifuged at 12000 × g at 4 °C for 20 minutes, and the supernatant was collected.

The virus was inactivated by S/D (solvent/detergent) method. The specific steps were as follows: After the collected supernatant was filtered by 0.45µm filter membrane, Tween-80 and tributyl phosphate were added successively, so that the final volume concentration of Tween-80 was 1%, and the final volume concentration of tributyl phosphate was 0.3%. After full mixing, the mixture was bathed in water at 25°C±2°C for 2 hours for viral inactivation. The venom obtained from viral inactivation was used to isolate coagulation factor X activating enzyme according to the steps listed in Table 3:

**Table 3 Summary of extraction methods of the coagulation factor X activating enzyme**

| Method | Step | | | | |
|---|---|---|---|---|---|
| 1.1 | / | Anion exchange chromatography (QHP) | Ultrafiltration | Cation exchange chromatography (Capto SP ImpRs) | / |
| 1.2 | / | Cation exchange chromatography (CM Beads 6FF) | Ultrafiltration | Anion exchange chromatography (NenoGel 50Q) | / |
| 1.3 | Size exclusion chromatograp hy | Anion exchange chromatography(QHP) | Ultrafiltration | Cation exchange chromatography (Capto SP ImpRs) | / |
| 1.4 | Size exclusion chromatograp hy | Cation exchange chromatography (Capto SP ImpRs) | Ultrafiltration | Cation exchange chromatography (CM Beads 6FF) | / |
| 1.5 | Size exclusion chromatograp hy | Anion exchange chromatography(Neno Gel 50Q) | Ultrafiltration | Cation exchange chromatography (Capto SP ImpRs) | / |
| 1.6 | / | Anion exchange chromatography(QHP) | Ultrafiltration | Cation exchange chromatography (Capto SP ImpRs) | Size exclusion chromatog raphy |
| 1.7 | Size exclusion chromatograp hy | Anion exchange chromatography(QHP) | Ultrafiltration | Cation exchange chromatography (Capto SP ImpRs) | Size exclusion chromatog raphy |
| 1.8 | Size exclusion chromatograp hy | Anion exchange chromatography(Neno Gel 50Q) | Ultrafiltration | Cation exchange chromatography (Capto SP ImpRs) | Size exclusion chromatog raphy |
| 1.9 | Size exclusion chromatograp hy | Anion exchange chromatography(QHP) | Ultrafiltration | Cation exchange chromatography (NanoGel -50SP) | Size exclusion chromatog raphy |
| 1.10 | Size exclusion chromatograp hy | Anion exchange chromatography(QHP) | Ultrafiltration | Cation exchange chromatography (CM Beads 6FF) | Size exclusion chromatog raphy |
| 1.11 | Size exclusion chromatograp hy | Anion exchange chromatography(QHP) | Ultrafiltration | Cation exchange chromatography (Capto SP ImpRs) | Hydropho bic chromatog raphy |
| 1.12 | Size exclusion chromatograp hy | Anion exchange chromatography(QHP) | Ultrafiltration | Cation exchange chromatography (NanoGel -50SP) | Hydropho bic chromatog raphy |
| 1.13 | Size exclusion chromatograp hy | Anion exchange chromatography(QHP) | Ultrafiltration | Cation exchange chromatography (Capto SP ImpRs) | Heparin affinity chromatog raphy |
| 1.14 | Heparin affinity chromatograp hy | Anion exchange chromatography(QHP) | Ultrafiltration | Cation exchange chromatography (Capto SP ImpRs) | / |
| 1.15 | Heparin affinity chromatograp hy | Anion exchange chromatography(QHP) | Ultrafiltration | Cation exchange chromatography (Capto SP ImpRs) | Size exclusio n chromat ography |
| 1.16 | Hydrophobic chromatograp hy | Anion exchange chromatography(Neno Gel 50Q) | Ultrafiltration | Cation exchange chromatography (Capto SP ImpRs) | / |
| 1.17 | Hydrophobic chromatograp hy | Anion exchange chromatography(Neno Gel 50Q) | Ultrafiltration | Cation exchange chromatography (Capto SP ImpRs) | Size exclusion chromatog raphy |

The specific steps were as follows:

### 1.1 Anion exchange chromatography-cation exchange chromatography

### (1) Anion exchange chromatography

The Q Sepharose HP column was equilibrated with 20mMTris-HCl (pH 8.5) buffer for 3 column volumes.

The snake venom after viral inactivation was loaded at a flow rate of 90cm/h. After sample loading, 20mM Tris-HCl (pH8.5) solution was used to wash impurities to the baseline at a flow rate of 90cm/h. The impurity was washed with 20mM Tris-HCl-0.2M NaCl (pH8.5) solution at a flow rate of 90cm/ h.

After washing, 20%-100% 20mM Tris-HCl-0.4M NaCl (pH8.5) solution was used to elute for 10 column volumes at a flow rate of 90cm/h. When the conductivity rose to 10ms/cm, the target peak began to appear, and the components corresponding to the target peak were collected. Components corresponding to the target peak were stopped to be collected when the target peak dropped to the baseline.

### (2) Ultrafiltration and concentration

The components corresponding to the target peaks collected after anion chromatography were concentrated 10 times by 30kD ultrafiltration membrane. The concentrate was then equal-volume exchanged with a 20mM phosphate buffer (pH 6.8).

### (3) Cation exchange chromatography

Capto SP ImpRes chromatographic column was equilibrated with 10mM (pH 6.8) phosphoric phosphate buffer for 3 column volumes, and then the components obtained after ultrafiltration were loaded at a flow rate of 60cm/h. After sample loading, 10mM phosphate buffer was used to re-equilibrate the chromatographic column to the baseline at a flow rate of 60cm/h.

After equilibration was completed, 0-100% solution of 10mM phosphate buffer +0.4M NaCl (pH 6.8) was used to linearly elute for 5 column volumes at a flow rate of 60cm/h. When the conductivity rose to 10±1ms/cm and UV showed an obvious inflection point, the target peak was collected. When the conductivity rose to 18±1ms/cm and the UV dropped to the next peak to rise, the collection was stopped.

### 1.2 Cation exchange chromatography-anion exchange chromatography

### (1) Cation exchange chromatography

CM Beads 6FF chromatographic columns were equilibrated with 10mM (pH 6.8) phosphate buffer for 3 column volumes, and then the components after viral inactivation were loaded at a flow rate of 60cm/h. After sample loading, 10mM phosphate buffer was used to re-equilibrate the chromatographic column to the baseline at a flow rate of 60cm/h.

After equilibration was completed, 0-100% solution of 10mM phosphate buffer +0.4M NaCl (pH 6.8) was used to linearly elute for 5 column volumes at a flow rate of 60cm/h, and components corresponding to the target peak were collected.

### (2) Ultrafiltration

The components corresponding to the target peaks collected after cationic chromatography were concentrated 10 times by 30kD ultrafiltration membrane. The concentrate was then equal-volume exchanged with 20mM Tris-HCl buffer (pH 8.5).

### (3) Anion exchange chromatography

NenoGel 50Q column was equilibrated with 20mMTris-HCl (pH8.5) buffer for 3 column volumes before use. The components obtained after ultrafiltration were loaded at a flow rate of 90cm/h. After sample loading, 20mMTris-HCl(pH8.5) buffer was used to re-equilibrate e to the baseline at a flow rate of 90cm/h. 20mM Tris-HCl-0.2M NaCl(pH8.5) solution was used to wash impurity at a flow rate of 90cm/ hour.

After washing, 20%-100% solution of 20mM Tris-HCl-0.4M NaCl(pH8.5) was used to elute for 10 column volumes at a flow rate of 90cm/h. When the conductivity rose to 10ms/cm, the target peak began to appear, and the components corresponding to the target peak were collected. Components corresponding to the target peak were stopped to be collected when the target peak dropped to the baseline.

### 1.3 Size exclusion chromatography-Anion exchange chromatography-Cation exchange chromatography

The snake venom after viral inactivation was first subjected to size exclusion chromatography under the following conditions: the G25 chromatographic column (≥2CV) was fully equilibrate at a flow rate of 60cm/ h with 0.02mol/L Tris-HCl (pH8.5) solution. The snake venom after viral inactivation was loaded at 20%-30% column volume each time, and washed with equilibrium solution after sampling. When the ultraviolet absorption value (UV value) rose to 1mAU, the collection of the target liquid was started, and the collection was stopped when the UV value dropped to 250±50 mAU.

The collected components were sequentially subjected to anion exchange chromatography, ultrafiltration and cation exchange chromatography according to steps (1) - (3) of Method 1.1, and the components corresponding to the target peak were collected.

### 1.4 Size exclusion chromatography-Cation exchange chromatography-Cation exchange chromatography

### (1) Size exclusion Chromatography

The snake venom after viral inactivation was subjected to size exclusion chromatography according to method 1.3.

### (2) Cation exchange chromatography

Capto SP ImpRes chromatographic column was equilibrated with 10mM (pH 6.8) phosphate buffer for 3 column volumes, and then the components obtained by Size exclusion Chromatography were loaded at a flow rate of 60cm/h. After sample loading, 10mM phosphate buffer was used to re-equilibrate the chromatographic column to the baseline at a flow rate of 60cm/h.

After equilibration was completed, 0-100% solution of 10mM phosphate buffer +0.4M NaCl (pH 6.8) was used to linearly elute for 5 column volumes at a flow rate of 60cm/h, and components corresponding to the target peak were collected.

### (3) Ultrafiltration

The components corresponding to the target peaks collected after cation chromatography were concentrated 10 times by 30kD ultrafiltration membrane. The concentrate was then equal-volume exchanged with 10mM phosphate buffer (pH 6.8).

### (4) Cation exchange chromatography

The components obtained after ultrafiltration were subjected to cation exchange chromatography with CM Beads 6FF: CM Beads 6FF chromatographic columns were equilibrated with 10mM (pH 6.8) phosphate buffer for 3 column volumes, and then the components obtained after ultrafiltration were loaded at a flow rate of 60cm/h. After sample loading, 10mM phosphate buffer was used to re-equilibrate the chromatographic column to the baseline at a flow rate of 60cm/h.

After equilibration was completed, 0-100% solution of 10mM phosphate buffer +0.4M NaCl (pH 6.8) was used to linearly elute for 5 column volumes at a flow rate of 60cm/h, and components corresponding to the target peak were collected.

### 1.5 Size exclusion chromatography-Anion exchange chromatography-Cation exchange chromatography

The snake venom after viral inactivation was purified according to Method 1.3 except that the chromatography column of anion exchange chromatography was a NenoGel 50Q column.

### 1.6 Anion exchange chromatography-Cation exchange chromatography-Size exclusion chromatography

The snake venom after viral inactivation were sequentially subjected to anion exchange chromatography, ultrafiltration and cation exchange chromatography according to the steps of Method 1.1, and then the corresponding components were collected.

Then, the collected components were subjected to size exclusion chromatography again under the following conditions: The Sephacry1 S-200HR column was fully equilibrated with 50mM (pH6.8) phosphate buffer for 3 column volumes before use and then the sample was loaded. The loading volume was controlled at 5% of the size exclusion column volume. The chromatographic flow rate was 20cm/h, and the components corresponding to the target peak were collected.

### 1.7 Size exclusion chromatography-Anion exchange chromatography-Cation exchange chromatography-Size exclusion chromatography

Size exclusion chromatography, anion exchange chromatography, ultrafiltration and cation exchange chromatography were sequentially performed according to the steps in Method 1.3 and the corresponding components were collected.

Then, the collected components were subjected to size exclusion chromatography under the following conditions: The Sephacry1 S-200HR column was fully equilibrated with 50mM (pH6.8) phosphate buffer for 3 column volumes before use and then the sample was loaded. The loading volume was controlled at 5% of the size exclusion column volume. The chromatographic flow rate was 20cm/h, and the components corresponding to the target peak were collected.

### 1.8 Size exclusion chromatography-Anion exchange chromatography-Cation exchange chromatography-Size exclusion chromatography

The snake venom after viral inactivation was purified according to Method 1.7 except that the chromatography column of anion exchange chromatography was NenoGel 50Q column.

### 1.9 Size exclusion chromatography-Anion exchange chromatography-Cation exchange chromatography-Size exclusion chromatography

The snake venom after viral inactivation was purified according to Method 1.7 except that the chromatography column of cation exchange chromatography was NenoGel 50SP column.

### 1.10 Size exclusion chromatography-Anion exchange chromatography-Cation exchange chromatography-Size exclusion chromatography

The snake venom after viral inactivation was purified according to Method 1.7 except that the chromatography column of cation exchange chromatography was CM Beads 6FF column.

### 1.11 Size exclusion chromatography-Anion exchange chromatography-Cation exchange chromatography-Hydrophobic chromatography

Size exclusion chromatography, anion exchange chromatography, ultrafiltration and cation exchange chromatography were performed sequentially according to the steps of Method 1.3, the corresponding components were collected and subjected to hydrophobic chromatography under the following conditions: The conductivity of the sample was adjusted to 140±5ms/cm; Butyl Bestarose HP column was equilibrated with 20mM phosphate buffer+2M NaCl (pH 7.0) for 3 column volumes before use and then the sample was loaded at a flow rate of 60cm/h. After sample loading, 20 mM phosphate buffer +2M NaCl pH7.0 solution was used to re-equilibrate the chromatographic column to the baseline at a flow rate of 60cm/h.

After equilibration was completed, 0-100% solution of 20mM (pH 6.8) phosphate buffer was used to linearly elute for 5 column volumes at a flow rate of 60cm/h, and components corresponding to the target peak were collected.

### 1.12 Size exclusion chromatography-Anion exchange chromatography-Cation exchange chromatography-Hydrophobic chromatography.

Size exclusion chromatography, anion exchange chromatography, ultrafiltration, cation exchange chromatography, and hydrophobic chromatography were subjected sequentially according to the steps of Method 1.11, and the corresponding components were collected, except that the chromatography column of cation exchange chromatography was NenoGel 50SP column.

### 1.13 Size exclusion chromatography-Anion exchange chromatography-Cation exchange chromatography-Heparin affinity chromatography

Size exclusion chromatography, anion exchange chromatography, ultrafiltration and cation exchange chromatography were subjected sequentially according to the steps of Method 1.3, and the components were collected, and subjected to heparin affinity chromatography, under the following conditions: The Capto heaparin chromatographic column was fully equilibrated with 20mM Tris-HCl (pH 8.0) buffer solution for the 3 column volumes and then the sample was loaded at a flow rate of 60cm/h. After sample loading, the chromatographic column was re-equilibrated with 20mM Tris-HCl (pH 8.0) buffer to the baseline at a flow rate of 60cm/h.

After equilibration was completed, 0-100% solution of 20mM Tris-HCl +1 M NaCl (pH 8.0) was used to linearly elute for 5 column volumes at a flow rate of 60cm/h, and components corresponding to the target peak were collected.

### 1.14 Heparin affinity chromatography-Anion exchange chromatography-Cation exchange chromatography

(1) The snake venom after viral inactivation was subjected to heparin affinity chromatography under the following conditions:
   The Capto heaparin chromatographic column was fully equilibrated with 20mM Tris-HCl (pH 8.0) buffer solution for the 3 column volumes and then the sample was loaded at a flow rate of 60cm/h. After sample loading, the chromatographic column was re-equilibrated with 20mM Tris-HCl (pH 8.0) buffer to the baseline at a flow rate of 60cm/h. After equilibration was completed, 0-100% solution of 20mM Tris-HCl +1M NaCl (pH 8.0) was used to linearly elute for 5 column volumes at a flow rate of 60cm/h, and components corresponding to the target peak were collected.
(2) Ultrafiltration

The components corresponding to the target peaks collected after heparin affinity chromatography were concentrated 10 times by 30kD ultrafiltration membrane. The concentrate was then equal-volume exchanged with 20mM Tris-HCl buffer (pH 8.5).

The components obtained after heparin affinity chromatography were sequentially performed by anion exchange chromatography, ultrafiltration and cation exchange chromatography according to the steps of Method 1.1, and then the corresponding components were collected.

### 1.15 Heparin affinity chromatography-Anion exchange chromatography-Cation exchange chromatography-Size exclusion chromatography

Heparin affinity chromatography, anion exchange chromatography, ultrafiltration and cation exchange chromatography were sequentially performed according to the steps in Method 1.14 and the corresponding components were collected. Then the collected components were subjected to size exclusion chromatography under the following conditions: The Sephacry1 S-200HR column was fully equilibrated with 50mM (pH6.8) phosphate buffer for 3 column volumes before use and then the sample was loaded. The loading volume was controlled at 5% of the size exclusion column volume. The chromatographic flow rate was 20cm/h, and the components corresponding to the target peak were collected.

### 1.16 Hydrophobic chromatography-Anion exchange chromatography-Cation exchange chromatography

(1) The snake venom after viral inactivation was subjected to hydrophobic chromatography under the following conditions: The conductivity of the sample was adjusted to 80±5ms/cm; Butyl Bestarose HP column was equilibrated with 20mM phosphate buffer+1M NaCl (pH 7.0) for 3 column volumes before use and then the sample was loaded at a flow rate of 60cm/h. After sample loading, the chromatographic column was re-equilibrated with 20mM phosphate buffer +1M NaCl pH7.0 to the baseline at a flow rate of 60cm/h. During this period, the flow-through was collected as the target peak.
(2) Ultrafiltration concentration.

The components corresponding to the target peaks collected after hydrophobic chromatography were concentrated 10 times by 30kD ultrafiltration membrane. The concentrate was then equal-volume exchanged with 20mM Tris-HCl buffer (pH 8.5).

The components obtained after Ultrafiltration were sequentially subjected to anion exchange chromatography, ultrafiltration, and cation exchange chromatography according to the steps of Method 1.1, and then the corresponding components were collected.

### 1.17 Hydrophobic chromatography-Anion exchange chromatography-Cation exchange chromatography-Size exclusion chromatography

Hydrophobic chromatography, ultrafiltration, anion exchange chromatography, ultrafiltration and cation exchange chromatography were sequentially performed according to the steps in Method 1.16 and the corresponding components were collected. Then, the collected components were subjected to size exclusion chromatography under the following conditions: The Sephacry1 S-200HR column was fully equilibrated with 50mM (pH6.8) phosphate buffer for 3 column volumes before use and then the sample was loaded. The loading volume was controlled at 5% of the size exclusion column volume. The chromatographic flow rate was 20cm/h, and the components corresponding to the target peak were collected.

### 2. Methods of detection and results

### (1) Method of detection

The coagulation factor X activating enzyme obtained by methods 1.1-1.17 was detected according to the following methods:
Detection of protein yield: The total protein concentration of snake venom during feeding was detected by BCA method, and the total protein mass was calculated according to the volume of the feeding liquid. The protein concentration of the purified coagulation factor X activating enzyme was detected by Folin phenol method (Lowry method), the second method of the determination of protein content of the third General Rule 0731 of the 2015 edition of Chinese Pharmacopoeia, and the volume was measured to obtain the total purified protein mass. The yield of the product was obtained by comparison with the total protein mass at the time of feeding.

Detection of protein purity: The purity was detected by High performance liquid chromatography (SEC-HPLC) based on size exclusion principle.

### (2) Detection results

The detection results of the coagulation factor X activating enzyme obtained by methods 1.1-1.17 were shown in Table 4.

**Table 4 The detection results of the coagulation factor X activating enzyme**

| Method | SEC-HPLC (%) | Protein yield (%) |
|---|---|---|
| 1.1 | 97.75 | 4.96 |
| 1.2 | 41.08 | / |
| 1.3 | 98.29 | 4.21 |
| 1.4 | 68.29 | / |
| 1.5 | 98.06 | 5.35 |
| 1.6 | 100 | 3.96 |
| 1.7 | 100 | 3.22 |
| 1.8 | 100 | 4.01 |
| 1.9 | 100 | 3.85 |
| 1.10 | 99.52 | 3.13 |
| 1.11 | 11.76 | 1.53 |
| 1.12 | 13.33 | 1.61 |
| 1.13 | 94.76 | 2.20 |
| 1.14 | 98.78 | 5.92 |
| 1.15 | 100 | 3.84 |
| 1.16 | 98.66 | 3.52 |
| 1.17 | 100 | 3.24 |

According to the above results, it can be concluded that:
(1) For the extraction of the coagulation factor X activating enzyme from the Russell's viper, if the extraction was carried out sequentially with one anion exchange chromatography and one cation exchange chromatography (Method 1.1), the purity of the final product could be achieved to 97.75% measured by SEC-HPLC, which was comparable to other methods in the prior art, and the yield of the product was as high as 4.96%, which was significantly higher than other methods in the prior art (e.g., Examples 5.2-5.3 of CN109943554A , the yield was 3.05%-3.49% after anion exchange chromatography and CHT purification).

However, if anion chromatography and cation chromatography were sequentially exchanged (Method 1.2) or two cation exchange chromatography were performed (Method 1.4), the purity of the final product measured by SEC-HPLC was only 41.08% and 68.29%, which was much lower than that of method 1.1.

These results indicated that the sequential combination of anion exchange chromatography and cation exchange chromatography was necessary to obtain high purity and high yield of coagulation factor X activating enzyme from the Russell's viper, and only one anion exchange chromatography and one cation exchange chromatography were required to obtain the desired high purity product without further purification steps.
(2) On the basis of the sequential combination of anion exchange chromatography and cation exchange chromatography, purification steps such as size exclusion chromatography, hydrophobic chromatography, or heparin affinity chromatography were performed before anion exchange chromatography(methods 1.3, 1.5, 1.14, 1.16), and the final product could maintain high purity (more than 98% measured by SEC-HPLC), and the product yield was still reached 3.52% to 5.92%, which was superior to the methods in the prior art (e.g., Examples 6-9 of CN109943554A, after purification by anion exchange chromatography, CHT and size exclusion chromatography, the yield was 2.69-2.82%).
(3) On the basis of the sequential combination of anion exchange chromatography and cation exchange chromatography, the cation exchange chromatography was followed by size exclusion chromatography (methods 1.6-1.10, 1.15, 1.17), which could increase the purity of the final product to 100%. Although there was a decrease in the yield (3.13%-4.01%), which may be due to excessive purification steps, however, the yield of the method of the present application was better than that of the prior art (e.g., Examples 6-9 of CN109943554A , after purification by anion exchange chromatography, CHT and size exclusion chromatography, the yield was 2.69-2.81%) under the condition that the number of purification steps was the same (method 1.6), and even the present application had an additional chromatography step (methods 1.7-1.10, 1.15, 1.17).

However, in the case of performing cation exchange chromatography followed by hydrophobic chromatography or heparin affinity chromatography (1.11, 1.12, 1.13), despite the addition of one purification step, the purity of the final product measured by SEC-HPLC decreased (11.76%, 13.33%, 94.76%) and the yield of the product also decreased to different degrees (1.53%-2.20%).

The above results indicate that on the basis of the sequential combinations comprising one anion exchange chromatography and one cation exchange chromatography, size exclusion chromatography steps may be added to further improve purity, or other purification steps may not be performed any more.

In summary, the method of the present application for isolating coagulation factor X activating enzyme from snake venom of the Russell's viper is capable of improving the yield of the product while ensuring high purity of the final product, which can greatly reduce the cost of production and be more suitable for large-scale production.

### Example 7. Structural characterization and activity determination of the coagulation factor X activating enzyme of the Russell's viper

The coagulation factor X activating enzyme samples purified by methods 1.1, 1.6 and 1.7 of Example 6 were selected for further structural characterization and activity determination.
1. Further confirmation of the amino acid sequence of coagulation factor X activating enzyme.
   The coagulation factor X activating enzyme was taken and further identified by mass spectrometry and the results showed that the amino acid sequence of the coagulation factor X activating enzyme was completely consistent with that of Example 3 (data not shown).
2. Analysis and identification the glycosylation modifications at each N-glycosylation site in coagulation factor X activating enzyme

Coagulation factor X activating enzyme was taken and centrifuged at 13000rpm for 10min; 400µl of 8M urea solution was added and centrifuged at 13000rpm for 10min; DTT solution at a final concentration of 10mM was added, after reaction at 56°C for 1h, IAM (iodoacetamide) solution at a final concentration of 30 mM was added, and the reaction was carried out in the dark at room temperature for 40min, and DTT solution at a final concentration of 10mM was again added to neutralize excess IAM solution; 350µl of 50 mM ammonium bicarbonate solution was added and centrifuged at 13000rpm for 10min, the lower layer solution was discard, and this step was repeated twice; trypsin was added at the ratio of 1:50 and reacted at 37°C for 2 hours, then trypsin was added again according to the above-mentioned enzyme dosage and reacted at 37°C overnight; formic acid solution was added at 1:20 ratio to terminate the enzyme digestion reaction; sample was taken into EP tube and ultrafiltration tube was washed twice and constant volume.

40µl of sample solution was precisely measured and injected to a liquid chromatography-mass spectrometer for analysis:

### Chromatographic conditions:

Column is ACQUITY UPLC^{®} CSHTM C18, 1.7µm, 2.1*150mm Column; column temperature: 40°C, sample temperature: 7°C, flow rate: 0.2ml/min, detection wavelength: 214nm; loading: 10µl, mobile phase A: 0.05% FA+0.05% TFA/H₂O, mobile phaseB:0.05% FA+0.05% TFA /(90% acetonitrile+10%H₂O).

The gradient elution procedure was shown in Table 5:

**Table 5 Gradient elution procedure**

| Time (min) | A% | B% |
|---|---|---|
| 0.00 | 98 | 2 |
| 3.00 | 98 | 2 |
| 55.00 | 90.5 | 9.5 |
| 105.00 | 78 | 22 |
| 120.00 | 58 | 42 |
| 125.00 | 2 | 98 |
| 130.00 | 2 | 98 |
| 130.01 | 98 | 2 |
| 150.00 | 98 | 2 |

### Mass Spectrometry Detection:

Analytical duration: 120 min, detection mode: positive ion, parent ion scan range: 300-2000 m/z, primary mass spectral resolution: 70,000 at m/z 200, secondary mass spectral resolution: 17,500 at m/z 200.

The identification results showed that among the three coagulation factor X activating enzyme samples, there were high glycosylation level at 6 glycosylation sites of the three peptide chains, and various N-glycan modifications were found at each site. It should be noted that although the sequence lengths of γ chain and β chain are close and both have only one N-glycosylation site, in general, the glycosylation modification at the Asn24 glycosylation site of the former is more complex than that at the Asn 59 site of the latter, and the types of N-glycan are more diverse (data not shown).

The results of analysis of the major N-glycotypes and their relative content at the 4 sites of the α chain (Asn 28, Asn 69, Asn 163, Asn 183) were shown in Table 6.

**Table 6 The major N-glycotypes and their relative content at each position of the α chain of the sample**

| Position | Glycotype | Relative content (%) | | |
|---|---|---|---|---|
| | | Method 1.1 | Method 1.6 | Method 1.7 |
| Asn 28 | A2BG2S2 | 28.89% | 25.03% | 25.58% |
| | FA2BG2S2 | 36.34% | 37.58% | 34.88% |
| | F2A2BG2S2 | 20.18% | 23.61% | 20.60% |
| | A3BG3S3 | 8.50% | 6.50% | 8.58% |
| Asn 69 | FA2S2 | 8.44% | 7.24% | 6.46% |
| | A2S2 | 7.72% | 6.56% | 6.83% |
| | FA3G2S2 | 11.63% | 13.79% | 9.81% |
| | A3G2S2 | 28.99% | 26.76% | 22.94% |
| | A4G3S3 | 18.96% | 15.10% | 19.46% |
| | A3S3 | 18.48% | 16.31% | 19.21% |
| Asn 163 | FA2G2S2 | 5.63% | 4.89% | 5.15% |
| | A2BG2S2 | 9.50% | 8.99% | 9.44% |
| | FA2BG2S2 | 19.80% | 21.54% | 20.19% |
| | F2A2BG2S2 | 15.04% | 20.12% | 16.58% |
| | A3G3S3 | 8.91% | 7.18% | 8.87% |
| | FA3G3S3 | 8.72% | 8.45% | 8.79% |
| | A3BG3S3 | 7.78% | 7.95% | 8.35% |
| | FA3BG3S3 | 5.84% | 7.10% | 6.56% |
| Asn 183 | A2G2S1 | 9.66% | 9.85% | 9.70% |
| | A2G2S1M4 | 5.29% | 5.59% | 5.58% |
| | A2BG1S1 | 13.44% | 12.54% | 13.20% |
| | FA2BG1S1 | 7.41% | 7.88% | 7.20% |
| | A2BG2S2 | 15.33% | 15.59% | 14.87% |
| | FA2BG2S2 | 8.21% | 9.62% | 8.12% |
| | M5 | 6.23% | 4.45% | 5.22% |

The results of analysis of the major N-glycotypes and their relative content at Asn 59 site of the β chain were shown in Table 7:

**Table 7 The major N-glycotypes and their relative content at each position of the β chain of the sample**

| Position | Glycotype | Relative content (%) | | |
|---|---|---|---|---|
| | | Method 1.1 | Method 1.6 | Method 1.7 |
| Asn 59 | A2BG2S2 | 23.13% | 19.01% | 22.31% |
| | FA2BG2S2 | 32.72% | 33.50% | 32.14% |
| | F2A2BG2S2 | 21.55% | 26.49% | 23.10% |
| | A3BG3S3 | 6.20% | 5.18% | 6.07% |
| | A2BG2S1 | 1.13% | 1.23% | 1.38% |
| | FA3G3S2 | 0.91% | 0.91% | 0.95% |

The results of analysis of the major N-glycotypes and their relative content at Asn 24 site of the γ chain were shown in Table 8:

**Table 8 The major N-glycotypes and their relative content at each position of the γ chain of the sample**

| Position | Glycotype | Relative content (%) | | |
|---|---|---|---|---|
| | | Method 1.1 | Method 1.6 | Method 1.7 |
| | A2BG1S1 | 7.70% | 6.79% | 7.62% |
| | FA2BG1S1 | 24.37% | 27.11% | 24.64% |
| | FA2BG2S1 | 5.13% | 6.31% | 5.97% |
| | A2BG2S2 | 13.59% | 11.63% | 13.65% |
| Asn 24 | FA2BG2S2 | 26.16% | 27.55% | 25.48% |
| | F2A2BG2S2 | 6.14% | 7.26% | 6.78% |
| | FA3G3S2 | 0.98% | 1.05% | 0.96% |
| | FA3BG3S3 | 1.65% | 1.05% | 1.42% |
| | A3BG2S2 | 1.54% | 0.97% | 1.26% |
| | A3BG3S3 | 2.24% | 1.53% | 2.05% |

### 3. Detection of coagulation factor X activating enzyme Activity

Three samples of the coagulation factor X activating enzyme described above were tested for enzyme activity by reference to the method of "Biobottom Color Continuous Rate Assay of coagulation factor X activating enzyme Activity of Russell's viper"(Chinese Journal of Medicine, 2007, Vol. 16, No. 18). The results of the assay showed that the specific activities of the coagulation factor X activating enzyme obtained by methods 1.1, 1.6 and 1.7 of the present application were 37589.2 U/mg, 38556.5 U/mg and 37992.8 U/mg, respectively.

## Claims

1. A coagulation factor X activating enzyme, wherein the coagulation factor X activating enzyme comprises three polypeptide chains: α, β, and γ, the amino acid sequence of the α chain is set forth in SEQ ID NO: 8, the amino acid sequence of the β chain is set forth in SEQ ID NO: 10, and the amino acid sequence of the γ chain is set forth in SEQ ID NO: 12.

2. The coagulation factor X activating enzyme of claim 1, wherein the α, β, and/or γ polypeptide chain has glycosylation modifications.

3. The coagulation factor X activating enzyme of claim 2, wherein at least one amino acid residue of the amino acid sequence of the α, β, and/or γ polypeptide chain is covalently linked to an N-glycan.

4. The coagulation factor X activating enzyme of claim 3, wherein the amino acid residue is asparagine (Asn) residue.

5. The coagulation factor X activating enzyme of claim 4, wherein the asparagine (Asn) residue site is selected from one or more of the following:
α chain: Asn28, Asn69, Asn163, Asn183;
β chain: Asn59; and/or
γ chain: Asn24.

6. The coagulation factor X activating enzyme of claim 5, wherein
N-glycans at the Asn28 site in the α chain comprise A2BG2S2, FA2BG2S2, F2A2BG2S2 and A3BG3S3; and/or,
N-glycans at the Asn69 site in the α chain comprise FA2S2, A2S2, FA3G2S2, A3G2S2, A4G3S3 andA3S3; and/or,
N-glycans at the Asn163 site in the α chain comprise FA2G2S2, A2BG2S2, FA2BG2S2, F2A2BG2S2, A3G3S3, FA3G3S3, A3BG3S3 and FA3BG3S3; and/or,
N-glycans at the Asn183 site in the α chain comprise A2G2S1, A2G2S1M4, A2BG1S1, FA2BG1S1, A2BG2S2, FA2BG2S2 and M5; and/or,
N-glycans at the Asn59 site in the β chain comprise A2BG2S2, FA2BG2S2, F2A2BG2S2, A3BG3S3, and further comprise N-glycans containing terminal galactose, preferably, the N-glycans containing terminal galactose comprise A2BG2S1, FA3G3S2, etc., and/or,
N-glycans at the Asn24 site in the γ chain comprise A2BG1S1, FA2BG1S1, FA2BG2S1, A2BG2S2, FA2BG2S2, F2A2BG2S2, and further comprise triantennary N-glycan or tetraantennary N-glycan containing fucosyl and/or acetyl sialic acid, preferably the triantennary N-glycan or tetraantennary N-glycan comprise FA3G3S2, FA3BG3S3, A3BG2S2, A3BG3S3, etc..

7. The coagulation factor X activating enzyme of claim 6, wherein:
N-glycans at the Asn28 site in the α chain comprise:
A2BG2S2 with a relative content of 22% to 32%, preferably 25% to 30%,
FA2BG2S2 with a relative content of 30% to 40%, preferably 34% to 38%,
F2A2BG2S2 with a relative content of 18% to 28%, preferably 20% to 25%, and
A3BG3S3 with a relative content of 5% to 10%, preferably 6% to 9%;
and/or,
N-glycans at the Asn69 site in the α chain comprise:
FA2S2 with a relative content of 5% to 10%, preferably 6% to 9%,
A2S2 with a relative content of 5% to 10%, preferably 6% to 8%,
FA3G2S2 with a relative content of 7% to 20%, preferably 9% to 15%,
A3G2S2 with a relative content of 20% to 30%, preferably 22% to 29%,
A4G3S3 with a relative content of 10% to 25%, preferably 15% to 20%, and
A3S3 with a relative content of 15% to 30%, preferably 16% to 20%;
and/or,
N-glycans at the Asn163 site in the α chain comprise:
FA2G2S2 with a relative content of 3% to 8%, preferably 4% to 6%,
A2BG2S2 with a relative content of 6% to 12%, preferably 8% to 10%,
FA2BG2S2 with a relative content of 15% to 25%, preferably 19% to 22%,
F2A2BG2S2 with a relative content of 10% to 25%, preferably 15% to 20%,
A3G3S3 with a relative content of 5% to 10%, preferably 7% to 9%,
FA3G3S3 with a relative content of 5% to 10%, preferably 8% to 9%,
A3BG3S3 with a relative content of 5% to 10%, preferably 7% to 9%; and
FA3BG3S3 with a relative content of 3% to 10%, preferably 5% to 8%;
and/or,
N-glycans at the Asn183 site in the α chain comprise:
A2G2S1 with a relative content of 5% to 15%, preferably 9% to 10%,
A2G2S1M4 with a relative content of 4% to 8%, preferably 5% to 6%,
A2BG1S1 with a relative content of 10% to 17%, preferably 12% to 14%,
FA2BG1S1 with a relative content of 5% to 10%, preferably 7% to 8%,
A2BG2S2 with a relative content of 10% to 20%, preferably 14% to 16%,
FA2BG2S2 with a relative content of 6% to 12%, preferably 8% to 10%; and
M5 with a relative content of 3% to 8%, preferably 4% to 7%;
and/or,
N-glycans at the Asn59 site in the β chain comprise:
A2BG2S2 with a relative content of 15% to 25%, preferably 19% to 24%,
FA2BG2S2 with a relative content of 30% to 40%, preferably 32% to 34%,
F2A2BG2S2 with a relative content of 20% to 30%, preferably 21% to 27%,
A3BG3S3 with a relative content of 3% to 10%, preferably 5% to 7%, and
N-glycans with terminal galactose in a relative content of 1% to 5%, preferably 2% to 4%
and/or,
N-glycans at the Asn24 site in the γ chain comprise:
A2BG1S1 with a relative content of 4% to 10%, preferably 6% to 8%,
FA2BG1S1 with a relative content of 20% to 30%, preferably 24% to 28%,
FA2BG2S1 with a relative content of 3% to 8%, preferably 5% to 7%,
A2BG2S2 with a relative content of 9% to 18%, preferably 11% to 14%,
FA2BG2S2 with a relative content of 20% to 30%, preferably 25% to 28%,
F2A2BG2S2 with a relative content of 4% to 10%, preferably 6% to 8%, and
triantennary N-glycan or tetraantennary N-glycan containing fucosyl and/or acetyl sialic acid with a relative content of 5% to 12%, preferably 6% to 10%;
wherein the relative content of N-glycans refers to the percentage of the total number of the coagulation factor X activating enzyme molecules containing the particular N-glycans at a certain site to the total number of all coagulation factor X activating enzyme molecules.

8. The coagulation factor X activating enzyme of any one of claims 1-7, wherein the coagulation factor X activating enzyme is derived from Russell's viper (Daboia russelii siamensis), preferably the coagulation factor X activating enzyme is isolated from Russell's viper venom or produced by genetic engineering.

9. A pharmaceutical composition, wherein the pharmaceutical composition comprises the coagulation factor X activating enzyme of any one of claims 1-8 and a pharmaceutically acceptable carrier.

10. A method of stopping bleeding or treatment of a bleeding disorder, wherein the method comprises administering to a subject in need thereof a therapeutically effective amount of the coagulation factor X activating enzyme of any one of claims 1-8 or a pharmaceutical composition of claim 9.

11. Use of the coagulation factor X activating enzyme of any one of claims 1 to 8 or a pharmaceutical composition of claim 9 in the manufacture of a medicament for stopping bleeding or treatment of the bleeding disorder.

12. The method of claim 10 or the use of claim 11, wherein the bleeding disorder comprises surgical wound bleeding, an internal hemorrhagic disease, a hereditary hemorrhagic disease, or a coagulation system disease; preferably the coagulation system disease comprises hemophilia; more preferably the hemophilia is a hemophilia with inhibitors; and more preferably the hemophilia is hemophilia A or hemophilia B.

13. An isolated nucleic acid molecule encoding the coagulation factor X activating enzyme of any one of claims 1-8.

14. A vector comprising the nucleic acid molecule of claim 13.

15. An isolated host cell, wherein the isolated host cell comprises the coagulation factor X activating enzyme of any one of claims 1-8, the nucleic acid molecule of claim 13, or the vector of claim 14.

16. A method for the preparation of the coagulation factor activating enzyme of any one of claims 1-8, wherein the method comprises the following steps:
a) culturing the host cell of claim 15 under conditions that can effectively express the coagulation factor X activating enzyme;
b) obtaining the expressed coagulation factor X activating enzyme from the host cell.

17. A method for glycosylation modification of the coagulation factor X activating enzyme of any one of claims 1-8, wherein the method is to make a genetically engineering host cell to produce the coagulation factor X activating enzyme having a predetermined N-glycosylation modification, and/or treating the coagulation factor X activating enzyme with glycosylation-related enzymes such that the coagulation factor X activating enzyme has a predetermined N-glycosylation modification.

18. A method for preparing the coagulation factor X activating enzyme of any one of claims 1 to 8, wherein the method comprises the following steps:
(a) obtaining the stock solution of the Russell's viper venom;
(b) isolating and purifying the stock solution of venom to obtain the coagulation factor activating enzyme of any one of claims 1-8.

19. A method for isolating a coagulation factor X activating enzyme, wherein the method comprises the following steps in turn:
(a) obtaining a sample of the stock solution of the Russell's viper venom;
(b) subjecting the sample to anion exchange chromatography;
(c) after anion exchange chromatography purification, subjecting the sample to cation exchange chromatography;
(d) obtaining the coagulation factor X activating enzyme.

20. The method of claim 19, wherein both anion exchange chromatography and cation exchange chromatography are carried out only once.

21. The method of claim 19 or 20, wherein no further purification steps are carried out after cation exchange chromatography, or size exclusion chromatography step is further included after cation exchange chromatography and no further purification steps are carried out.

22. The method of any one of claims 19-21, wherein the packing medium in the anion exchange chromatography column is agarose or polymer;
preferably, the chromatography column packed with agarose medium is selected from Sepharose H. P., Sepharose F. F., Capto, or Diamond, more preferably Q Sepharose H. P., Q Sepharose F.F, Capto Q, EDAE-Sepharose FF, Diamond Q or Diamond MIX-A;
preferably, the packed medium is polymer which is polystyrene/stilbene, polyacrylate, or diethylaminoethyl; preferably, the chromatography column packed with polymer medium is NenoGel 50 Q, UniGel 65 Q HC or UniGel 30DEAE.

23. The method of any one of claims 19-22, wherein the elution buffer of the anion exchange chromatography is Tris-HCl buffer containing NaCl with a pH of 8.0 to 8.6, wherein the concentration of Tris-HCl buffer is from 10 mM to 30 mM and the concentration of NaCl is from 0.1M to 0.5M;
preferably, the pH of Tris-HCl buffer is 8.5, and the concentration of Tris-HCl buffer is 20 mM; and the concentration of NaCl is 0.4M;
preferably, the elution flow rate of the anion exchange column chromatography is from 30 cm/hour to 150 cm/hour, preferably from 80 cm/hour to 120 cm/hour, more preferably 90 cm/hour.

24. The method of any one of claims 19-23, wherein the packed medium of the cation exchange chromatography column is agarose or polymer;
preferably, the chromatography column packed with agarose medium is selected from Capto SP ImpRes, CM Beads 6FF, or Diamond MMC;
preferably, the polymer is preferably polystyrene/stilbene; and preferably, the chromatography column packed with polymer medium is NenoGel 50 SP.

25. The method of any one of claims 19-24, wherein the elution buffer of the cation exchange chromatography is phosphate buffer containing NaCl with a pH of 6.5 to 7.5, wherein the concentration of phosphate buffer is from 10 mM to 30 mM, and the concentration of NaCl is from 0.2M to 0.6M;
preferably, the pH of phosphate buffer is 6.8, the concentration of phosphate buffer is 10 mM, and the concentration of NaCl is 0.4 M;
preferably, the elution flow rate of the cation exchange column chromatography is from 30 cm/hour to 150 cm/hour, preferably 40 cm/hour to 100 cm/hour, more preferably 60 cm/hour.

26. The method of any one of claims 19-25, wherein further comprising size exclusion chromatography, heparin affinity chromatography, and/or hydrophobic chromatography before anion exchange chromatography.

27. The method of claim 26, wherein the packed medium of size exclusion chromatography column is gel filtration media, preferably Sephacryl, Superdex, or Sephadex, more preferably Sephacryl S-200 HR, Superdex75pg, Superdex200pg or SephadexG25,
preferably, the elution buffer of the size exclusion chromatography is Tris-HCl buffer with a pH of from 8.0 to 8.6, and the concentration of Tris-HCl buffer is from 10 mM to 30 mM; preferably the pH of Tris-HCl buffer is 8.5, and the concentration is 20 mM;
preferably, the elution flow rate of the size exclusion chromatography is from 30 cm/hour to 150 cm/hour, preferably from 40 cm/hour to 120 cm/hour, more preferably 60 cm/hour.

28. The method of claim 26, wherein the packed medium of the heparin affinity chromatography column is agarose medium; preferably Capto heparin, Heparin Berpharose F. F., Heparin Sepharose 6 F. F., or Heparin Sepharose H. P.;
preferably, the elution buffer of the heparin affinity chromatography is Tris-HCl buffer containing NaCl with a pH of from 7.6 to 8.4, wherein the concentration of Tris-HCl buffer is from 10 mM to 30 mM and the concentration of NaCl is 0.5M to 1.5M;
preferably, the pH of Tris-HCl buffer is 8.0, the concentration of Tris-HCl buffer is 20 mM; and the concentration of NaCl is 1.0M;
preferably, the elution flow rate of the heparin affinity chromatography is from 30 cm/hour to 150 cm/hour, preferably from 40 cm/hour to 120 cm/hour, more preferably 60 cm/hour.

29. The method of claim 26, wherein the packed medium of the hydrophobic chromatography column is agarose; preferably Butyl Bestarose HP;
preferably, the elution buffer of the hydrophobic chromatography is phosphate buffer containing NaCl with a pH of from 6.5 to 7.5, wherein the concentration of phosphate buffer is from 10 mM to 30 mM, and the concentration of NaCl is from 0.5M to 2.5M;
preferably, the pH of the phosphate buffer is 7.0, the concentration of phosphate buffer is 20 mM; and the concentration of NaCl is 1.0 M;
preferably, the elution flow rate of the hydrophobic chromatography is from 30 cm/hour to 150 cm/hour, preferably from 40 cm/hour to 120 cm/hour, more preferably 60 cm/hour.

30. The method of any one of claims 19-29, wherein further comprising size exclusion chromatography after the cation exchange chromatography;
preferably, the packed medium of the size exclusion chromatography is gel filtration media, preferably Sephacryl, Superdex, or Sephadex, more preferably Sephacryl S-200 HR, Superdex75pg, Superdex200pg or SephadexG25;
preferably, the elution buffer of the size exclusion chromatography is phosphate buffer with a pH of from 6.5 to 7.5, and the concentration of phosphate buffer is from 40 mM to 80 mM, more preferably the pH of phosphate buffer is 6.8, and the concentration of phosphate buffer is 50 mM;
preferably, the elution flow rate of the size exclusion chromatography is from 5 cm/hour to 80 cm/hour, preferably from 10 cm/hour to 35 cm/hour, more preferably 20 cm/hour.

31. The method of any one of claims 19-30, wherein further comprising ultrafiltration after anion exchange chromatography and before cation exchange chromatography;
preferably, the specific steps of ultrafiltration are: collecting the components corresponding to the target peak of anion exchange chromatography and concentrating 5-20 times with an ultrafiltration membrane, and then exchanging the buffer of the components corresponding to the target peak with 10 mM-30mM phosphate buffer, wherein the pH of the phosphate buffer is from 6.5 to 7.5, and the molecular weight cut-off of ultrafiltration membrane is from 10kD to 50kD;
more preferably, the concentration of phosphate buffer is 20mM, the pH of phosphate buffer is 6.8, the molecular weight cut-off of ultrafiltration membrane is 30kD, and the concentration ratio is 10 times.

32. The method of any one of claims 19-31, wherein after obtaining a sample of the stock solution of snake venom and before anion chromatography purification, further comprising the step of treating the stock solution of the snake venom with viral inactivation; preferably the viral inactivation treatment is carried out by the S/D (solvent/detergent) method.

33. The method of any one of claims 19-32, wherein the coagulation factor X activating enzyme is the coagulation factor X activating enzyme of any one of claims 1-8.
